# EUROPEAN PATENT APPLICATION

(11) **EP 2 806 276 A1**
(43) Date of publication of application: **26.11.2014**
(21) Application number: 13168970.5
(22) Date of filing: 23.05.2013
(51) Int. Cl.: G01N 33/68

(54) **Method for post mortem diagnosis of Alzheimer's Disease (AD)**

(71) Applicant: Red Bull GmbH, 5330 Fuschl am See (AT)
(72) Inventor: Lubec, Barbara, 1220 Wien (AT); Lubec, Gert, 1220 Wien (AT)
(74) Representative: Sonn & Partner Patentanwälte

(57) **Abstract**

Disclosed is a method for diagnosing Alzheimer's Disease (AD) post mortem, wherein the amount of at least one receptor complex, selected from the group consisting of nicotinic acetylcholine receptor complex 4, nicotinic acetylcholine receptor complex 7, dopamine receptor complex D1 and dopamine receptor complex D2, is measured in a brain sample of a patient and diagnosing AD, if the amount of the complex in the sample is increased compared to normal, non-AD levels.

SEQUENCE LISTING

<110>  Red Bull GmbH

<120>  Method for post mortem diagnosis of Alzheimer's Disease (AD)

<130>  R 63566

<160>  5

<170>  PatentIn version 3.5

<210>  1
<211>  446
<212>  PRT
<213>  Homo sapiens

<400>  1

Met Arg Thr Leu Asn Thr Ser Ala Met Asp Gly Thr Gly Leu Val Val
1               5                   10                  15

Glu Arg Asp Phe Ser Val Arg Ile Leu Thr Ala Cys Phe Leu Ser Leu
            20                  25                  30

Leu Ile Leu Ser Thr Leu Leu Gly Asn Thr Leu Val Cys Ala Ala Val
        35                  40                  45

Ile Arg Phe Arg His Leu Arg Ser Lys Val Thr Asn Phe Phe Val Ile
    50                  55                  60

Ser Leu Ala Val Ser Asp Leu Leu Val Ala Val Leu Val Met Pro Trp
65                  70                  75                  80

Lys Ala Val Ala Glu Ile Ala Gly Phe Trp Pro Phe Gly Ser Phe Cys
                85                  90                  95

Asn Ile Trp Val Ala Phe Asp Ile Met Cys Ser Thr Ala Ser Ile Leu
            100                 105                 110

Asn Leu Cys Val Ile Ser Val Asp Arg Tyr Trp Ala Ile Ser Ser Pro
        115                 120                 125

Phe Arg Tyr Glu Arg Lys Met Thr Pro Lys Ala Ala Phe Ile Leu Ile
    130                 135                 140

Ser Val Ala Trp Thr Leu Ser Val Leu Ile Ser Phe Ile Pro Val Gln
145                 150                 155                 160

Leu Ser Trp His Lys Ala Lys Pro Thr Ser Pro Ser Asp Gly Asn Ala
                165                 170                 175

Thr Ser Leu Ala Glu Thr Ile Asp Asn Cys Asp Ser Ser Leu Ser Arg
            180                 185                 190

Thr Tyr Ala Ile Ser Ser Ser Val Ile Ser Phe Tyr Ile Pro Val Ala
        195                 200                 205

Ile Met Ile Val Thr Tyr Thr Arg Ile Tyr Arg Ile Ala Gln Lys Gln
    210                 215                 220

Ile Arg Arg Ile Ala Ala Leu Glu Arg Ala Ala Val His Ala Lys Asn
225                 230                 235                 240

Cys Gln Thr Thr Thr Gly Asn Gly Lys Pro Val Glu Cys Ser Gln Pro
                245                 250                 255

Glu Ser Ser Phe Lys Met Ser Phe Lys Arg Glu Thr Lys Val Leu Lys
            260                 265                 270

Thr Leu Ser Val Ile Met Gly Val Phe Val Cys Cys Trp Leu Pro Phe
        275                 280                 285

Phe Ile Leu Asn Cys Ile Leu Pro Phe Cys Gly Ser Gly Glu Thr Gln
    290                 295                 300

Pro Phe Cys Ile Asp Ser Asn Thr Phe Asp Val Phe Val Trp Phe Gly
305                 310                 315                 320

Trp Ala Asn Ser Ser Leu Asn Pro Ile Ile Tyr Ala Phe Asn Ala Asp
                325                 330                 335

Phe Arg Lys Ala Phe Ser Thr Leu Leu Gly Cys Tyr Arg Leu Cys Pro
            340                 345                 350

Ala Thr Asn Asn Ala Ile Glu Thr Val Ser Ile Asn Asn Asn Gly Ala
        355                 360                 365

Ala Met Phe Ser Ser His His Glu Pro Arg Gly Ser Ile Ser Lys Glu
    370                 375                 380

Cys Asn Leu Val Tyr Leu Ile Pro His Ala Val Gly Ser Ser Glu Asp
385                 390                 395                 400

Leu Lys Lys Glu Glu Ala Ala Gly Ile Ala Arg Pro Leu Glu Lys Leu
                405                 410                 415

Ser Pro Ala Leu Ser Val Ile Leu Asp Tyr Asp Thr Asp Val Ser Leu
            420                 425                 430

Glu Lys Ile Gln Pro Ile Thr Gln Asn Gly Gln His Pro Thr
        435                 440                 445

<210>  2
<211>  938
<212>  PRT
<213>  Homo sapiens

<400>  2

<210>  3
<211>  906
<212>  PRT
<213>  Homo sapiens

<400>  3

Met Gln His Ile Phe Ala Phe Phe Cys Thr Gly Phe Leu Gly Ala Val
1               5                   10                  15

Val Gly Ala Asn Phe Pro Asn Asn Ile Gln Ile Gly Gly Leu Phe Pro
            20                  25                  30

Asn Gln Gln Ser Gln Glu His Ala Ala Phe Arg Phe Ala Leu Ser Gln
        35                  40                  45

Leu Thr Glu Pro Pro Lys Leu Leu Pro Gln Ile Asp Ile Val Asn Ile
    50                  55                  60

Ser Asp Ser Phe Glu Met Thr Tyr Arg Phe Cys Ser Gln Phe Ser Lys
65                  70                  75                  80

Gly Val Tyr Ala Ile Phe Gly Phe Tyr Glu Arg Arg Thr Val Asn Met
                85                  90                  95

Leu Thr Ser Phe Cys Gly Ala Leu His Val Cys Phe Ile Thr Pro Ser
            100                 105                 110

Phe Pro Val Asp Thr Ser Asn Gln Phe Val Leu Gln Leu Arg Pro Glu
        115                 120                 125

Leu Gln Asp Ala Leu Ile Ser Ile Ile Asp His Tyr Lys Trp Gln Lys
    130                 135                 140

Phe Val Tyr Ile Tyr Asp Ala Asp Arg Gly Leu Ser Val Leu Gln Lys
145                 150                 155                 160

Val Leu Asp Thr Ala Ala Glu Lys Asn Trp Gln Val Thr Ala Val Asn
                165                 170                 175

Ile Leu Thr Thr Thr Glu Glu Gly Tyr Arg Met Leu Phe Gln Asp Leu
            180                 185                 190

Glu Lys Lys Lys Glu Arg Leu Val Val Val Asp Cys Glu Ser Glu Arg
        195                 200                 205

Leu Asn Ala Ile Leu Gly Gln Ile Ile Lys Leu Glu Lys Asn Gly Ile
    210                 215                 220

Gly Tyr His Tyr Ile Leu Ala Asn Leu Gly Phe Met Asp Ile Asp Leu
225                 230                 235                 240

Asn Lys Phe Lys Glu Ser Gly Ala Asn Val Thr Gly Phe Gln Leu Val
                245                 250                 255

Asn Tyr Thr Asp Thr Ile Pro Ala Lys Ile Met Gln Gln Trp Lys Asn
            260                 265                 270

Ser Asp Ala Arg Asp His Thr Arg Val Asp Trp Lys Arg Pro Lys Tyr
        275                 280                 285

Thr Ser Ala Leu Thr Tyr Asp Gly Val Lys Val Met Ala Glu Ala Phe
    290                 295                 300

Gln Ser Leu Arg Arg Gln Arg Ile Asp Ile Ser Arg Arg Gly Asn Ala
305                 310                 315                 320

Gly Asp Cys Leu Ala Asn Pro Ala Val Pro Trp Gly Gln Gly Ile Asp
                325                 330                 335

Ile Gln Arg Ala Leu Gln Gln Val Arg Phe Glu Gly Leu Thr Gly Asn
            340                 345                 350

Val Gln Phe Asn Glu Lys Gly Arg Arg Thr Asn Tyr Thr Leu His Val
        355                 360                 365

Ile Glu Met Lys His Asp Gly Ile Arg Lys Ile Gly Tyr Trp Asn Glu
    370                 375                 380

Asp Asp Lys Phe Val Pro Ala Ala Thr Asp Ala Gln Ala Gly Gly Asp
385                 390                 395                 400

Asn Ser Ser Val Gln Asn Arg Thr Tyr Ile Val Thr Thr Ile Leu Glu
                405                 410                 415

Asp Pro Tyr Val Met Leu Lys Lys Asn Ala Asn Gln Phe Glu Gly Asn
            420                 425                 430

Asp Arg Tyr Glu Gly Tyr Cys Val Glu Leu Ala Ala Glu Ile Ala Lys
        435                 440                 445

His Val Gly Tyr Ser Tyr Arg Leu Glu Ile Val Ser Asp Gly Lys Tyr
    450                 455                 460

Gly Ala Arg Asp Pro Asp Thr Lys Ala Trp Asn Gly Met Val Gly Glu
465                 470                 475                 480

Leu Val Tyr Gly Arg Ala Asp Val Ala Val Ala Pro Leu Thr Ile Thr
                485                 490                 495

Leu Val Arg Glu Glu Val Ile Asp Phe Ser Lys Pro Phe Met Ser Leu
        500                 505                 510

Gly Ile Ser Ile Met Ile Lys Lys Pro Gln Lys Ser Lys Pro Gly Val
    515                 520                 525

Phe Ser Phe Leu Asp Pro Leu Ala Tyr Glu Ile Trp Met Cys Ile Val
    530                 535                 540

Phe Ala Tyr Ile Gly Val Ser Val Val Leu Phe Leu Val Ser Arg Phe
545                 550                 555                 560

Ser Pro Tyr Glu Trp His Ser Glu Glu Phe Glu Glu Gly Arg Asp Gln
                565                 570                 575

Thr Thr Ser Asp Gln Ser Asn Glu Phe Gly Ile Phe Asn Ser Leu Trp
            580                 585                 590

Phe Ser Leu Gly Ala Phe Met Gln Gln Gly Cys Asp Ile Ser Pro Arg
        595                 600                 605

Ser Leu Ser Gly Arg Ile Val Gly Gly Val Trp Trp Phe Phe Thr Leu
    610                 615                 620

Ile Ile Ile Ser Ser Tyr Thr Ala Asn Leu Ala Ala Phe Leu Thr Val
625                 630                 635                 640

Glu Arg Met Val Ser Pro Ile Glu Ser Ala Glu Asp Leu Ala Lys Gln
                645                 650                 655

Thr Glu Ile Ala Tyr Gly Thr Leu Glu Ala Gly Ser Thr Lys Glu Phe
            660                 665                 670

Phe Arg Arg Ser Lys Ile Ala Val Phe Glu Lys Met Trp Thr Tyr Met
        675                 680                 685

Lys Ser Ala Glu Pro Ser Val Phe Val Arg Thr Thr Glu Glu Gly Met
    690                 695                 700

Ile Arg Val Arg Lys Ser Lys Gly Lys Tyr Ala Tyr Leu Leu Glu Ser
705                 710                 715                 720

Thr Met Asn Glu Tyr Ile Glu Gln Arg Lys Pro Cys Asp Thr Met Lys
                725                 730                 735

Val Gly Gly Asn Leu Asp Ser Lys Gly Tyr Gly Ile Ala Thr Pro Lys
            740                 745                 750

Gly Ser Ala Leu Arg Asn Pro Val Asn Leu Ala Val Leu Lys Leu Asn
        755                 760                 765

Glu Gln Gly Leu Leu Asp Lys Leu Lys Asn Lys Trp Trp Tyr Asp Lys
    770                 775                 780

Gly Glu Cys Gly Ser Gly Gly Gly Asp Ser Lys Asp Lys Thr Ser Ala
785                 790                 795                 800

Leu Ser Leu Ser Asn Val Ala Gly Val Phe Tyr Ile Leu Ile Gly Gly
                805                 810                 815

Leu Gly Leu Ala Met Leu Val Ala Leu Ile Glu Phe Cys Tyr Lys Ser
            820                 825                 830

Arg Ser Glu Ser Lys Arg Met Lys Gly Phe Cys Leu Ile Pro Gln Gln
        835                 840                 845

Ser Ile Asn Glu Ala Ile Arg Thr Ser Thr Leu Pro Arg Asn Ser Gly
    850                 855                 860

Ala Gly Ala Ser Ser Gly Gly Ser Gly Glu Asn Gly Arg Val Val Ser
865                 870                 875                 880

His Asp Phe Pro Lys Ser Met Gln Ser Ile Pro Cys Met Ser His Ser
                885                 890                 895

Ser Gly Met Pro Leu Gly Ala Thr Gly Leu
            900                 905

<210>  4
<211>  883
<212>  PRT
<213>  Homo sapiens

<400>  4

<210> 5
<211> 902
<212> PRT
<213> Homo sapiens

<400> 5

Met Arg Ile Ile Ser Arg Gln Ile Val Leu Leu Phe Ser Gly Phe Trp
1               5                   10                  15

Gly Leu Ala Met Gly Ala Phe Pro Ser Ser Val Gln Ile Gly Gly Leu
            20                  25                  30

Phe Ile Arg Asn Thr Asp Gln Glu Tyr Thr Ala Phe Arg Leu Ala Ile
        35                  40                  45

Phe Leu His Asn Thr Ser Pro Asn Ala Ser Glu Ala Pro Phe Asn Leu
    50                  55                  60

Val Pro His Val Asp Asn Ile Glu Thr Ala Asn Ser Phe Ala Val Thr
65                  70                  75                  80

Asn Ala Phe Cys Ser Gln Tyr Ser Arg Gly Val Phe Ala Ile Phe Gly
                85                  90                  95

Leu Tyr Asp Lys Arg Ser Val His Thr Leu Thr Ser Phe Cys Ser Ala
            100                 105                 110

Leu His Ile Ser Leu Ile Thr Pro Ser Phe Pro Thr Glu Gly Glu Ser
        115                 120                 125

Gln Phe Val Leu Gln Leu Arg Pro Ser Leu Arg Gly Ala Leu Leu Ser
    130                 135                 140

Leu Leu Asp His Tyr Glu Trp Asn Cys Phe Val Phe Leu Tyr Asp Thr
145                 150                 155                 160

Asp Arg Gly Tyr Ser Ile Leu Gln Ala Ile Met Glu Lys Ala Gly Gln
                165                 170                 175

Asn Gly Trp His Val Ser Ala Ile Cys Val Glu Asn Phe Asn Asp Val
            180                 185                 190

Ser Tyr Arg Gln Leu Leu Glu Glu Leu Asp Arg Arg Gln Glu Lys Lys
        195                 200                 205

Phe Val Ile Asp Cys Glu Ile Glu Arg Leu Gln Asn Ile Leu Glu Gln
    210                 215                 220

Ile Val Ser Val Gly Lys His Val Lys Gly Tyr His Tyr Ile Ile Ala
225                 230                 235                 240

Asn Leu Gly Phe Lys Asp Ile Ser Leu Glu Arg Phe Ile His Gly Gly
                245                 250                 255

Ala Asn Val Thr Gly Phe Gln Leu Val Asp Phe Asn Thr Pro Met Val
            260                 265                 270

Ile Lys Leu Met Asp Arg Trp Lys Lys Leu Asp Gln Arg Glu Tyr Pro
        275                 280                 285

Gly Ser Glu Thr Pro Pro Lys Tyr Thr Ser Ala Leu Thr Tyr Asp Gly
    290                 295                 300

Val Leu Val Met Ala Glu Thr Phe Arg Ser Leu Arg Arg Gln Lys Ile
305                 310                 315                 320

Asp Ile Ser Arg Arg Gly Asn Ala Gly Asp Cys Leu Ala Asn Pro Ala
                325                 330                 335

Ala Pro Trp Gly Gln Gly Ile Asp Met Glu Arg Thr Leu Lys Gln Val
            340                 345                 350

Arg Ile Gln Gly Leu Thr Gly Asn Val Gln Phe Asp His Tyr Gly Arg
        355                 360                 365

Arg Val Asn Tyr Thr Met Asp Val Phe Glu Leu Lys Ser Thr Gly Pro
    370                 375                 380

Arg Lys Val Gly Tyr Trp Asn Asp Met Asp Lys Leu Val Leu Ile Gln
385                 390                 395                 400

Asp Val Pro Thr Leu Gly Asn Asp Thr Ala Ala Ile Glu Asn Arg Thr
                405                 410                 415

Val Val Val Thr Thr Ile Met Glu Ser Pro Tyr Val Met Tyr Lys Lys
            420                 425                 430

Asn His Glu Met Phe Glu Gly Asn Asp Lys Tyr Glu Gly Tyr Cys Val
        435                 440                 445

Asp Leu Ala Ser Glu Ile Ala Lys His Ile Gly Ile Lys Tyr Lys Ile
    450                 455                 460

Ala Ile Val Pro Asp Gly Lys Tyr Gly Ala Arg Asp Ala Asp Thr Lys
465                 470                 475                 480

Ile Trp Asn Gly Met Val Gly Glu Leu Val Tyr Gly Lys Ala Glu Ile
                485                 490                 495

Ala Ile Ala Pro Leu Thr Ile Thr Leu Val Arg Glu Glu Val Ile Asp
            500                 505                 510

Phe Ser Lys Pro Phe Met Ser Leu Gly Ile Ser Ile Met Ile Lys Lys
        515                 520                 525

Pro Gln Lys Ser Lys Pro Gly Val Phe Ser Phe Leu Asp Pro Leu Ala
    530                 535                 540

Tyr Glu Ile Trp Met Cys Ile Val Phe Ala Tyr Ile Gly Val Ser Val
545                 550                 555                 560

Val Leu Phe Leu Val Ser Arg Phe Ser Pro Tyr Glu Trp His Thr Glu
                565                 570                 575

Glu Pro Glu Asp Gly Lys Glu Gly Pro Ser Asp Gln Pro Pro Asn Glu
            580                 585                 590

Phe Gly Ile Phe Asn Ser Leu Trp Phe Ser Leu Gly Ala Phe Met Gln
        595                 600                 605

Gln Gly Cys Asp Ile Ser Pro Arg Ser Leu Ser Gly Arg Ile Val Gly
    610                 615                 620

Gly Val Trp Trp Phe Phe Thr Leu Ile Ile Ile Ser Ser Tyr Thr Ala
625                 630                 635                 640

Asn Leu Ala Ala Phe Leu Thr Val Glu Arg Met Val Ser Pro Ile Glu

                645                 650                 655

Ser Ala Glu Asp Leu Ala Lys Gln Thr Glu Ile Ala Tyr Gly Thr Leu
            660                 665                 670

Asp Ser Gly Ser Thr Lys Glu Phe Phe Arg Arg Ser Lys Ile Ala Val
        675                 680                 685

Tyr Glu Lys Met Trp Thr Tyr Met Arg Ser Ala Glu Pro Ser Val Phe
    690                 695                 700

Thr Arg Thr Thr Ala Glu Gly Val Ala Arg Val Arg Lys Ser Lys Gly
705                 710                 715                 720

Lys Phe Ala Phe Leu Leu Glu Ser Thr Met Asn Glu Tyr Ile Glu Gln
                725                 730                 735

Arg Lys Pro Cys Asp Thr Met Lys Val Gly Gly Asn Leu Asp Ser Lys
            740                 745                 750

Gly Tyr Gly Val Ala Thr Pro Lys Gly Ser Ser Leu Arg Thr Pro Val
        755                 760                 765

Asn Leu Ala Val Leu Lys Leu Ser Glu Ala Gly Val Leu Asp Lys Leu
    770                 775                 780

Lys Asn Lys Trp Trp Tyr Asp Lys Gly Glu Cys Gly Pro Lys Asp Ser
785                 790                 795                 800

Gly Ser Lys Asp Lys Thr Ser Ala Leu Ser Leu Ser Asn Val Ala Gly
                805                 810                 815

Val Phe Tyr Ile Leu Val Gly Gly Leu Gly Leu Ala Met Leu Val Ala
            820                 825                 830

Leu Ile Glu Phe Cys Tyr Lys Ser Arg Ala Glu Ala Lys Arg Met Lys
        835                 840                 845

Leu Thr Phe Ser Glu Ala Ile Arg Asn Lys Ala Arg Leu Ser Ile Thr
    850                 855                 860

Gly Ser Val Gly Glu Asn Gly Arg Val Leu Thr Pro Asp Cys Pro Lys
865                 870                 875                 880

Ala Val His Thr Gly Thr Ala Ile Arg Gln Ser Ser Gly Leu Ala Val
                885                 890                 895

Ile Ala Ser Asp Leu Pro
            900

## Description

The present invention relates to methods for diagnosing Alzheimer's Disease (AD).

AD is the most common form of dementia in elderly persons. It is a neurodegenerative disease marked by decline in memory and cognitive performance, including deterioration of language as well as defects in visual and motor coordination, and eventual death. AD is morphologically characterized by extracellular beta-amyloid (Aβ) plaque deposition, intraneuronal taupathology, neuronal cell death, beginning in the entorhinal cortex and later spreading to the neocortex, loss of certain types of receptors, vascular dysfunction and inflammatory processes.

Several lines of evidence point to a link between brain nicotinic acetylcholine receptors (nAChRs) and the development of AD. Biochemical analysis of brains of patients with AD reveals cholinergic deficits, i.e. an increase in butyrylcholinesterase, reduction in acetylcholine, and attenuated activity of cholinergic synthetic (choline acetyltransferase (ChAT)) and inactivating (acetyl choline esterase; AChE)) enzymes. The most vulnerable neurons in AD seem to be those expressing high levels of nAChRs, particularly those containing the α7 subunit and the numbers of nAChRs as well as some of their associated proteins are reduced in AD. In addition, not only have α7 nAChRs been found colocalized with plaques but α7 and α4 subunits are also positively correlated with neurons that accumulate Aβ.

The discovery that nicotine, a ligand acting at nAChRs, and its mimetics can protect neurons against Aβ toxicity is of interest, especially in view of the observation that nicotine also enhances cognition. Nicotinic AChRs play a particularly prominent role in nicotine protection. The protective effect is blocked by the nicotinic antagonists dihydro-β-erythroidine and mecamylamine. In addition to nicotine, donepezil and rivastigmine, AChE inhibitors currently used as treatments for mild or moderate AD under the brand names of Aricept and Exelon, also protect cultured neuroblastoma cells from the toxic effects of Aβ.

There is evidence that cholinesterase inhibitors can promote the release of glutamate from pyramidal neurons, possibly by increasing cortical ACh levels and subsequent activation of the AChRs. In addition, it has been demonstrated that uncoupling of the postsynaptic muscarinic M1 receptor from G-proteins can correlate with the loss of N-methyl-D-aspartate (NMDA) receptor density and protein kinase C (PKC) activity in post mortem frontal cortex of individuals with AD.

A selective serotonin 1A receptor (5-HT1_{A}R) molecular imaging probe was used in conjunction with positron emission tomography for quantification of 5-HT1_{A}R densities in brains of AD patients with mild cognitive impairment and controls. Brains of patients with AD had significantly decreased receptor densities in both, hippocampi and raphe nuclei. The glutamatergic neurons are situated in areas that are known to be affected in AD, and the initial damage is proposed to begin in pyramidal neurons in layers III and V of the neocortex and glutamate innervated cortical and hippocampal neurons.

Although it is known that AD is a synaptic disease involving various neurotransmitter systems, particularly of those where synaptic transmission is mediated by acetyl choline or glutamate, very little is known about qualitative and quantitative changes of neurotransmitter receptors in the AD brain. It was shown previously that cell membranes, carrying neurotransmitter receptors from the human postmortem brain, can be transplanted to frog oocytes where receptors will still be functional. Taking advantage of this fact, the properties of glutamate receptors (GluRs) from cerebral cortices of AD and non-AD brains have been studied and it has been found that oocytes injected with AD membranes acquired GluRs that have essentially the same functional properties as those of oocytes injected with membranes from non-AD brains. However, the amplitudes of the currents elicited by GluRs were always smaller in the oocytes injected with membranes from AD brains. Western blot analyses of the same membrane preparations used for the electrophysiological studies showed that AD membranes contained significantly fewer GluR2/3 subunit proteins. Furthermore, the corresponding mRNAs were also diminished in the AD brain. Therefore, the smaller amplitude of membrane currents elicited by Glu in oocytes injected with membranes from an AD brain is a consequence of a reduced number of GluRs in cell membranes transplanted from the AD brain.

AD is usually diagnosed clinically from the patient history, collateral history from relatives, and clinical observations, based on the presence of characteristic neurological and neuropsychological features and the absence of alternative conditions. Advanced medical imaging with computed tomography (CT) or magnetic resonance imaging (MRI), and with single photon emission computed tomography (SPECT) or positron emission tomography (PET) can be used to help exclude other cerebral pathology or subtypes of dementia. Moreover, it may predict conversion from prodromal stages (mild cognitive impairment) to AD. Assessment of intellectual functioning including memory testing can further characterise the state of the disease. However, definitive confirmation of AD is still only performed post-mortem when brain material is available and can be examined histologically by identification of the neurofibrillary tangles and abnormal amyloid plaque deposits. There is currently no biochemical diagnostic method available that could objectively confirm the histological analysis.

There is still an unmet need for a reliable diagnostic (post mortem) method for AD that applies objective biochemical criteria. It is an object of the present invention to provide such a method.

With respect to AD therapy, only five medications are currently used to treat the cognitive manifestations of AD: four are acetylcholinesterase inhibitors (tacrine, rivastigmine, galantamine and donepezil) and the other (memantine) is an NMDA receptor antagonist. No drug has an indication for delaying or halting the progression of the disease. There is still no cure for Alzheimer's disease; available treatments offer relatively small symptomatic benefit but remain palliative in nature.

Therefore, there is also an unmet need for treatment of AD. Accordingly, it is another object to provide means for treatment of AD.

Therefore, the present invention provides a method for diagnosing Alzheimer's Disease (AD) post mortem, wherein the amount of at least one receptor complex, selected from the group consisting of nicotinic acetylcholine receptor complex 4, nicotinic acetylcholine receptor complex 7, dopamine receptor complex D1 and dopamine receptor complex D2, is measured in a brain sample of a patient and diagnosing AD, if the amount of the complex in the sample is increased compared to normal, non-AD levels.

In addition, the present invention also provides a method for diagnosing Alzheimer's Disease (AD) in a male patient post mortem, wherein the amount of at least one receptor complex, selected from the group consisting of muscarinic acetylcholine receptor complex M1, α-amino-3-hydroxy-5-methyl-4-isoxazolepropionic acid (AMPA) receptor complex GluR1 and N-methyl-D-aspartate (NMDA) receptor complex NR1, is measured in a brain sample of a male patient and diagnosing AD, if the amount of the complex in the sample is increased compared to normal male, non-AD levels.

Previous work on brain receptor subunits has shown the importance of these signaling compounds for AD, although there is no evidence for changes of receptor complexes. Using a native gel-based immunochemical method to determine major brain receptor complex levels in brains of patients with AD, it was revealed by the present invention that a series of receptor complex level changes were forming a receptor complex pattern in cortical brain areas from patients with AD and controls that may lead to or parallel reveal deterioration of the physiological concerted action of receptor complexes.

The findings according to the present invention revealed a pattern of deranged receptor complex levels that may indicate deterioration of the concerted action of these receptors which in turn are indicative for cognitive deficits observed in patients with AD. It has to be emphasised that it is the receptor complexes that are the working units rather than the individual subunits (see also: Schwenk et al., Neuron 74 (2012), 621-633). According to the present invention, receptor deficits have been revealed that are also relevant for designing experimental therapies and indeed, specific pharmacological modulation of the abovementioned receptors is in the pharmaceutical repertoire and can also be provided by the present invention in view of the outcome of the present invention.

With the present invention, an objective biochemical tool is provided for diagnosing AD post mortem. This enables a significant advantage compared to the histological diagnosis currently available for AD that necessitates experienced and therefore still subjective analysis by histologists.

Preferably, the brain samples used according to the present invention are a temporal cortical sample. The samples should be taken as early as possible, e.g. within 12 h, preferably within 6 h, especially within 3 h post mortem. This enables an unbiased determination of receptor complexes in brain tissue according to the present invention.

Preferably, measuring the receptor complex according to the present invention includes one or more of the following steps: purification of the complex by density gradient purification, especially sucrose gradient purification, native gel electrophoresis, especially blue native polyacrylamide gel electrophoresis, immunoblotting, protein digestion, especially protein digestion with trypsin and/or chymotrypsin, and mass spectroscopy. In the example section, an optimised method is disclosed, however, modern biochemistry analysis allows various variations of the method according to the present invention for isolating and determination of receptor complexes that can easily be established for any specialised laboratory concerned.

As also shown in the example section, the receptor complex markers according to the present invention allow statistically significant diagnosis of AD by the determination of increased levels of specific receptor complexes present in the brain AD patients. Applied to a broad (serial) diagnostic testing it is preferred to have an increase of the amount of the complex according to the present invention of at least 30%, preferably at least 50%, more preferred at least 100%, especially at least 200%, is indicative for AD when compared to the level of the very complex in healthy non-AD persons.

A preferred embodiment of the present invention employs measuring the receptor complexes specifically in a range of a molecular weight of 200 to 1500 kDa, preferably of 300 to 1000 kDa, especially of 400 to 800 kDa, in native gel electrophoresis. It is shown in the example section of the present application that this molecular size range offers a specifically suitable window for analysing receptor complexes according to the present invention.

Preferably, detection and measuring the receptor complex includes identification of the receptor complexes by receptor binding substances, preferably antibodies, especially polyclonal antibodies, against Dopamine receptor D1, Dopamine Receptor D2, Nicotinic Acetylcholine Receptor alpha4 and Nicotinic Acetylcholine Receptor alpha7; or by antibodies, especially polyclonal antibodies, against GluR1, muscarinic receptor M1 and NR1. Such antibodies are well available in the art and can be purchased commercially. These primary antibodies may be detected and quantitated by various means, e.g. by suitable secondary antibodies that are labelled, e.g. secondary anti-goat, -rabbit, -mouse, -rat, -sheep, etc. antibodies, if the primary antibody was goat, rabbit, mouse, rat, sheep, etc., respectively. The secondary antibody may be labelled, e.g. by a chomogenic, fluorescent or luminescent label. Suitable labels include Fluorescein (FITC), 2,4-Dinitrophenol (DNP), myc Digoxigenin (DIG), tyrosine, biotin/streptavidin, nitrotyrosine biotin and dyes. e.g. tetramethylrhodamine, Texas Red, dansyl, Alexa Fluor 488, BODIPY FL, lucifer yellow and Alexa Fluor 405/Cascade Blue fluorophores, enzyme labels, such as horseradish peroxidase (HRP), alkaline phosphatase (AP), beta-galactosidase (GAL), glucose-6-phosphate dehydrogenase, beta-N-acetylglucosaminidase, β-glucuronidase, invertase, xanthine oxidase, firefly luciferase or glucose oxidase (GO).

According to a specific aspect, the present invention also refers to a kit for performing the method according to the present invention (i.e. for use in diagnosing AD) comprising
- detection means for at least one of nicotinic acetylcholine receptor complex 4, nicotinic acetylcholine receptor complex 7, dopamine receptor complex D1 and dopamine receptor complex D2, or at least one of muscarinic acetylcholine receptor complex M1, α-amino-3-hydroxy-5-methyl-4-isoxazolepropionic acid (AMPA) receptor complex GluR1 and N-methyl-D-aspartate (NMDA) receptor complex NR1, and
- a sample container.

The kit may further comprise necessary elements for individual, commercial and serial diagnostic testing, such as primary (as detection means for the complex(es)) and secondary antibodies as disclosed above, standards (e.g. positive and/or negative controls, quantitative standards, qualitative standards, comparative samples of known AD status (verified AD, verified healthy (=non-AD)), kit leaflet with instructions to use and quantitative information concerning the complex(es) to be determined. The components of the kits are preferably determined for use in human sample analysis and are therefore prepared by GMP and provided in a sterile form.

According to another aspect, the present invention provides a pharmaceutical preparation containing a D1 modulator and, optionally, a pharmaceutically acceptable excipient, for use in a method for treatment of AD.

The results according to the present invention not only enabled a reliable diagnostic method for AD, the biochemical teaching provided herewith also enables a new route of treating AD. The elevated levels of the receptor complexes according to the present invention can be functionally correlated to AD symptoms in cognitive performance and the pathological development thereof. Specifically increased levels of dopamine receptor complex D1 and dopamine receptor complex D2 offers pharmacological strategies for the treatment of AD by D1 modulators. Substances binding to D1 can be used for counteracting increased levels of dopamine receptor complex D1 and also - because of the high homology between D1 and D2 (and also D3, D4 and D5; i.e. "D1-like") - for dopamine receptor complex D2. The two receptor subtypes are highly homologous and very few ligands are selective between them. According to a specific embodiment, such D1 binding substances may be labelled by a positron-emitting radionuclide to form a radiotracer, typically isotopes with short half-lives such as carbon-11 (∼20 min), nitrogen-13 (∼10 min), oxygen-15 (∼2 min), fluorine-18 (∼110 min), or rubidum-82 (∼1.27 min). This also allows prae mortem diagnosis, see below.

Accordingly, it is preferred to select the D1 modulator from the group consisting of cis-(±)-1-(Aminomethyl)-3,4-dihydro-3-phenyl-1H-2-benzopyran-5,6-diol hydrochloride (A 68930 hydrochloride), (1R-cis)-1-(Aminomethyl)-3,4-dihydr-o-3-tricyclo[3.3.1.13,7]dec-1-yl-[1H]-2-benzopyran-5,6-diol hydrochloride (A 77636 hydrochloride), (R)-5,6,6a,7-Tetrahydro-6-methyl-4H--dibenzo[de,g]quinoline-10,11-diol hydrochloride ((R) - (-) -Apomorphine hydrochloride), (-) - (6aR, 12bR)-4,6,6a,7,8,12b-Hexahydro-7-methylindolo[4,3-a] phenanthridin (CY 208-243), (±)-trans-10,11-Dihydroxy-5,6,6a,7,8-,12b-hexahydrobenzo[a]phenanthridine hydrochloride (Dihydrexidine hydrochloride), 3,4-Dihydroxyphenethylamine hydrochloride (Dopamine hydrochloride), 6-Chloro-2,3,4,5-tetrahydro-1-(4-hy-droxyphenyl)-1H-3-benzazepine-7,8-diol hydrochloride (Fenoldopam hydrochloride), (N)-1-(2-Nitrophenyl)ethylcarboxy-3-,4-dihydroxyphenethylamine (NPEC-caged-dopamine), (±)-1-Phenyl-2,3,4,5-tetrahydro-(1H)-3-benzazepine-7,8-diol hydrobromide (SKF 38393 hydrobromide), 2,3,4,5-Tetrahydro-1-phenyl-3-(2-propenyl)-1H-3-benzazepine-7,8-diol hydrobromide (SKF 77434 hydrobromide), (±)-6-Chloro-2,3,4,5-tetrahydro-1-phenyl-1H-3-benzazepine hydrobromide (SKF 81297 hydrobromide), 6-Chloro-2,3,4,5-tetrahydro-1-(3-methylphenyl)-3-(2-propenyl)-1H-3-benzazepine-7,8-di-ol hydrobromide (SKF (83822 hydrobromide), 6-Chloro-2,3,4,5-tetrahydro-3-methy-1-1-(3-methylphenyl)-1H-3-benzazepine-7,8-diol (SKF 83959 hydrobromide), 6,7,8,9,14,15-Hexahydro-7-methyl-5H--indolo[3,2-f][3]benzazecine (LE 300), (R)-(+)-7-Chloro-8-hydroxy-3-methyl-1-phenyl-2,3,4,5-tetrahydro-1H-3-benzazepine hydrochloride (SCH 23390 hydrochloride), (6aS-trans)-11-Chloro-6,6a,7,8,9,13-b-hexahydro-7-methyl-5H-benzo[d]naphth[2,1-b]azepin-12-ol hydrobromide (SCH 39166 hydrobromide), 8-Bromo-2,3,4,5-tetrahydro-3-methyl-5-phenyl-1H-3-benzazepin-7-ol hydrobromide (SKF 83566 hydrobromide), and pharmaceutically acceptable free, salt and ester forms of such D1 modulators.

Suitable administration routes are all routes that may be applied on a routine basis for the treatment of cognitive diseases, e.g. orally, nasally, rectally, intravenously (especially if resorption disorders are present), etc.. Suitable pharmaceutically acceptable excipients are those known and already used for the specific compounds, for the intended administration route and for the intended dosage form (tablet, capsule, ampule, drink, powder inhalation, etc.).

According to another aspect, the present invention also provides an in vivo diagnosing of AD by PET. Accordingly, the present invention provides a method for diagnosing Alzheimer's Disease (AD), wherein a D1 modulator radiolabelled with a positron-emitting radionuclide is administered to a patient, the patient's brain is subjected to a Positron emission tomography (PET) and wherein an increased binding and localisation of the radiolabelled D1 modulator in the brain compared to a normal, non-AD person, is indicative of AD.

With the present invention, an increased binding/localisation of the D1 modulator is indicative of an increased presence of dopamine receptor complex D1 and dopamine receptor complex D2 in the brain which is indicative of AD. According to the post mortem aspect of the present invention, this is a clear biochemical diagnosis of AD. The PET embodiment of the present invention is the in vivo (prae mortem) equivalent of this teaching according to the present invention.

PET is an established method as a nuclear medical imaging technique that produces a three-dimensional image or picture of functional processes in the body. In PET, the PET system detects pairs of gamma rays emitted indirectly by a positron-emitting radionuclide (tracer), which is introduced into the body on a biologically active molecule (according to the present invention, a D1 modulator, i.e. a substance that binds specifically to D1 (and D2 (besides D3, D4 and D5); accordingly, these receptors can also be referred to as "D1-like receptors"). Three-dimensional images of tracer concentration within the body (according to the present invention: in the brain) are then constructed by computer analysis. In modern scanners, three dimensional imaging is often accomplished with the aid of a CT X-ray scan performed on the patient during the same session, in the same machine. Practical embodiments of the present method may therefore be designed according to the established technology. For example, the radionuclide is preferably selected from carbon-11, nitrogen-13, oxygen-15, fluorine-18, and rubidum-82.

Preferably, the patient's temporal cortex is subjected to PET. This is the brain region where the increase in dopamine receptor complex D1 and dopamine receptor complex D2 is specifically indicative.

Also brain PET is already an established method in neurology, neuropsychology/cognitive neuroscience and psychiatry. However, this has up to now been mainly established on the general assumption that areas of high radioactivity are associated with brain activity, since such brain PETs have been used to measure brain activity in general (e.g. by glucose turnover, etc.).

The present invention is further illustrated by the following examples and the figures, yet without being restricted thereto.
Figure 1-4: Arbitrary units of optical density of receptor complex levels that were not significantly different between controls and patients with AD.
Figure 5: Receptor complex levels different between male controls and male patients with AD expressed as arbitrary units of optical density.
Figure 6: Receptor complex levels different between both, males and females, controls and patients with AD.
Figure 7: Lining up of the receptor complexes for the determination of receptor complex composition. The high molecular weight material on gels is from glycosylation known to generate this pattern and this was not used for quantification.
Figure 8: (A) Full listing of correlation of receptor complexes in male controls, (B) in female controls.
Figure 9: (A) Full listing of correlation of receptor complexes in male patients with AD and (B) female patients with AD.
Figure 10: SDS-PAGE of the immunoprecipitate using a D1 receptor antibody. The D1 receptor along with a single band containing NR1, GluR1, 2 and 4 was identified by mass spectrometry.
Figure 11a to 11e: Representative mass spectra identifying D1 (340-AFSTLLGCYR-349), NR1 (770-QNVSLSILK-778), GluR1 (187-MLFQDLEK-194), 2 (871-K.EGYNVYGIESVK.I-882) and 4 (761-GSSLRTPVNLAVLK-774).

### Examples:

### Changes of several brain receptor complex levels in cerebral cortex of patients with AD

Although AD has been linked to defects in major brain receptors, work so far has been limited to the determination of receptor subunits or specific ligand binding studies. The availability of current technology allowing the determination and quantification of brain receptor complexes enabled to examine native receptor complex levels in brains of patients with AD in the course of the present invention.

Cortical tissue of controls (n=12 females, 12 males) and patients with AD (n=12 females and 12 males) at a short postmortem time of 3 hours, were kept frozen until biochemical analyses. Membrane proteins were extracted and subsequently enriched by ultracentrifugation using a sucrose gradient. Membrane proteins were run on native gels and were immunoblotted with antibodies against individual brain receptors.

Receptor complex levels were comparable for complexes containing GluR2, GluR3 and GluR4 as well as 5-HT1_{A}. Receptor complex levels containing muscarinic AChR M1, NR1 and GluR1 were significantly increased in male patients with AD. Nicotinic AChRs 4 and 7, dopamine receptors D1 and D2 were increased in males and females with AD.

### Materials and Methods

### Patients

### Case recruitment and characterization of human brain tissue

Case recruitment and autopsy were performed in accordance with guidelines effective at the Banner Sun Health Research Institute Brain Donation Program of Sun City, Arizona, USA (Beach et al., Cell Tissue Bank 9 (2008), 229-245). The required consent was obtained for all cases. The definite diagnosis of AD for all cases used in this study was based on the presence of neurofibrillary tangles and neuritic plaques in the hippocampal formation and neocortical areas and met the criteria of the National Institute on Aging (NIA) and the Consortium to Establish a Registry for AD (CERAD) (Mirra et al., Neurology 41 (1991), 479-486). Temporal cortex tissue from 24 AD cases (12 female, 12 male) and from 24 age-matched control subjects (12 female, 12 male) was evaluated for neurotransmitter receptor complexes (Table 1).

**Table 1: Case numbers, gender, age and Braak scores of samples used for the analyses**

| | **Case #** | **Gender** | **Age (years)** | **Break score** |
|---|---|---|---|---|
| **Control** | 1 | Female | 85 | II |
| | 2 | Female | 85 | II |
| | 3 | Female | 86 | III |
| | 4 | Female | 89 | III |
| | 5 | Female | 75 | III |
| | 6 | Female | 87 | IV |
| | 7 | Female | 82 | II |
| | 8 | Female | 95 | III |
| | 9 | Female | 87 | III |
| | 10 | Female | 82 | II |
| | 11 | Female | 78 | II |
| | 12 | Female | 84 | III |
| | 13 | Male | 81 | I |
| | 14 | Male | 82 | III |
| | 15 | Male | 74 | II |
| | 16 | Male | 78 | I |
| | 17 | Male | 86 | I |
| | 18 | Male | 89 | II |
| | 19 | Male | 81 | III |
| | 20 | Male | 80 | I |
| | 21 | Male | 91 | II |
| | 22 | Male | 82 | III |
| | 23 | Male | 73 | II |
| | 24 | Male | 78 | III |
| **AD** | 1 | Female | 82 | VI |
| | 2 | Female | 85 | VI |
| | 3 | Female | 79 | VI |
| | 4 | Female | 84 | VI |
| | 5 | Female | 85 | VI |
| | 6 | Female | 82 | VI |
| | 7 | Female | 82 | VI |
| | 8 | Female | 73 | VI |
| | 9 | Female | 78 | VI |
| | 10 | Female | 84 | VI |
| | 11 | Female | 78 | VI |
| | 12 | Female | 83 | VI |
| | 13 | Male | 80 | VI |
| | 14 | Male | 77 | VI |
| | 15 | Male | 87 | VI |
| | 16 | Male | 78 | VI |
| | 17 | Male | 77 | VI |
| | 18 | Male | 81 | VI |
| | 19 | Male | 70 | VI |
| | 20 | Male | 85 | VI |
| | 21 | Male | 85 | VI |
| | 22 | Male | 89 | VI |
| | 23 | Male | 72 | VI |
| | 24 | Male | 74 | VI |

### Biochemical analyses

### Sample preparation

48 temporal cortical samples from patients with AD and controls (12 male and 12 female controls and 12 male and 12 female samples with AD) were homogenized in ice-cold homogenization buffer (10 mM, HEPES, pH 7.5, 300 mM sucrose, one complete protease inhibitor tablet (Roche Molecular Biochemicals, Mannheim, Germany) per 50 mL) by Ultra-Turrax (IKA, Staufen, Germany). The homogenate was centrifuged for 10 min at 1,000 x g and the pellet was discarded. The supernatant was centrifuged at 50,000 x g for 30 min in an ultracentrifuge (Beckman Coulter Optima L-90K). Subsequently, the pellet was homogenized in 5 mL washing buffer (homogenization buffer without sucrose), kept on ice for 30 min and centrifuged at 50,000 x g for 30 min.

### Sucrose gradient ultracentrifugation for membrane fractionation

The plasma membrane purification procedures from the pellet was carried out as described previously, with slight modifications (Chen et al., Journal of neurochemistry 2006 Aug; 98(4): 1126-1140; Kang et al., Journal of proteome research 2008 Aug; 7(8): 3498-3506). Sucrose density gradient centrifugation solutions of 700 µL each of 69, 54, 45, 41 and 37% (w/v) were formed. Membrane pellets in 500 µL were resuspended in homogenization buffer, layered on top of the tubes that were filled with homogenization buffer. Samples were ultracentrifuged at 4°C at 70,000 x g for 3 h. After centrifugation, the 41% fraction from the sucrose interface was collected, diluted 10 times with homogenization buffer, and then ultracentrifuged at 4°C at 100,000 x g for 30 min. After discarding the supernatant, the pellet was stored at -80°C until use (Ghafari et al., Brain structure & function 2012 Apr; 217(2): 353-362).

### Blue native-polyacrylamide gel electrophoresis (BN-PAGE)

Membrane pellets from the 41% sucrose gradient ultracentrifugation fraction were solubilized in extraction buffer (1.5 M 6-aminocaproic acid, 300 mM Bis-Tris, pH 7.0) and 10% Triton X-100 (stock solution was added at a ratio of 1:4 to achieve final 2% Triton X-100 concentration) with vortexing every 10 min for 1 h. Following solubilization, samples were cleared by centrifugation at 20,000 x g for 60 min at 4°C. The protein content was estimated using the BCA protein assay kit (Pierce, Rockford, IL, USA). 50 µg of the membrane protein preparation were applied onto gels. 16 µL BN PAGE loading buffer (5% (w/v) Coomassie G250 in 750 mM 6-aminocaproic acid) were mixed with 100 µL of the membrane protein preparation and loaded onto the gel. BN-PAGE was performed in a PROTEAN II xi Cell (BioRad, Germany) using 4% stacking and 5-18% separating gel. The BN-PAGE gel buffer contained 500 mM 6-aminocaproic acid, 50 mM Bis-Tris, pH 7.0; the cathode buffer 50 mM Tricine, 15 mM Bis-Tris, 0.05% (w/v) Coomassie G250, pH 7.0; and the anode buffer 50 mM Bis-Tris, pH 7.0. The voltage was set to 50 V for 1 h, 75 V for 6 h, and was increased sequentially to 400 V (maximum current 15 mA/gel, maximum voltage 500 V) until the dye front reached the bottom of the gel (Ghafari et al., 2012). Native high molecular mass markers were obtained from Invitrogen (Carlsbad, CA, USA).

### Immunoblotting

Membrane proteins were transferred from BN-PAGE to PVDF membranes. After blocking of membranes for 1 h with 10% non-fat dry milk in 0.1% TBST (100 mM Tris-HCL, 150 mM NaCl, pH 7.5, 0.1% Tween 20), membranes were incubated with primary antibodies: rabbit against GluR1 (Abcam, ab31232, Cambridge, UK), rabbit against GluR2 (Abcam, ab52932, Cambridge, UK), rabbit against GluR3 (Abcam, ab87609, Cambridge, UK), rabbit against GluR4 (Abcam, ab109431, Cambridge, UK), rabbit against Dopamine receptor D1(Abcam, ab85608, Cambridge, UK), rabbit against Dopamine Receptor D2 (Millipore, AB5084P), rabbit against Nicotinic Acetylcholine Receptor alpha4 (Abcam, ab41172, Cambridge, UK), rabbit against Nicotinic Acetylcholine Receptor alpha7 (Abcam, ab10096, Cambridge, UK), rabbit against Muscarinic Receptor M1 (Abcam, ab75178, Cambridge, UK), rabbit against NMDAR1 (Abcam, ab28669, Cambridge, UK), rabbit against 5HT1A (GenScript, Piscataway, NJ, USA), and detected with horseradish peroxidase-conjugated anti-rabbit IgG (Abcam, ab6721, Cambridge, UK). Membranes were developed with the ECL Plus Western Blotting Detection System (GE Healthcare, Buckinghamshire, UK). Arbitrary optical densities of immunoreactive bands were measured by the Image J software program (http://rsb.info.nih.gov/ij/).

### Immunoprecipitation of Dopamin D1 receptor from human brain

In order to show interaction partners of the D1 receptor with other receptors immunoprecipitation was carried out on temporal cortex of human brain.

Total hippocampal membrane fractions (Ghafari et al., 2012) from temporal cortex were suspended in lysis buffer containing 1% Triton X100, 150 mM NaCl, 1 mM EDTA, 50 mM Tris-HCl (pH 8.0), 10 mM NaF, 10 mM Na₃VO₄ and protease inhibitor cocktail (Roche, Mannheim, Germany) on a rotation shaker for 1 h at 4 °C. After centrifugation at 15300 x g, at 4°C for 10 min, the supernatant was incubated with affinity purified rabbit antibody against Dopamin D1 receptor (D1DR, Santa Cruz, Santa Cruz, CA) and subsequently incubated with protein G agarose beads (GE Healthcare, Uppsala, Sweden) for 4 h at 4°C with gentle rotation. After five times of washing with the same lysis buffer, proteins bound were denatured with sample buffer containing 125 mM Tris (pH 6.8), 4% SDS, 20% glycerol, 10% betamercaptoethanol, 0.02% bromophenol blue at 95 °C for 3 min (Ghafari et al., Journal of proteome research 2012 Mar 2; 11(3): 1891-1896). Samples were loaded onto 10% SDS-polyacrylamide gels, electrophoresed, and subsequently transferred to PVDF membranes (Pall, Ann Harbor, MI). After blocking of membranes for 1 h with 5% nonfat dry milk in 0.1% TBST (100 mM Tris-HCL, 150 mM NaCl, pH 7.5, 0.1% Tween 20), membranes were incubated with diluted goat primary antibody against Dopamin D1 receptor (1:1000, Santa Cruz, Santa Cruz, CA) and detected with anti-goat IgG (1:5,000, Abcam, Cambridge, U.K.). Membranes were developed with the ECL Plus Western Blotting Detection System (GE Healthcare, Uppsala, Sweden).

### In-gel Digestion of Proteins and Peptides

Spots picked from the SDS gel that were recognised by the corresponding antibody against the Dopamin D1 receptor subunit were put into a 1.5 mL Eppendorf tube. Gel pieces were washed with 50 mM ammonium bicarbonate and then two times with washing buffer (50% 100 mM ammonium bicarbonate/50% acetonitrile) for 30 min each with vortexing. An aliquot of 100 µL of 100% acetonitrile was added to the tube to cover the gel pieces completely and the mixture was incubated for 10 min. Gel pieces were dried completely using a SpeedVac concentrator. Reduction of cysteine residues was carried out with a 10 mM dithiothreitol (DTT) solution in 100 mM ammonium bicarbonate pH 8.6 for 60 min at 56°C. After discarding the DTT solution the same volume of a 55 mM iodoacetamide (IAA) solution in 100 mM ammonium bicarbonate buffer pH 8.6 was added and incubated in darkness for 45 min at 25°C to achieve alkylation of cysteine residues. The IAA solution was replaced by washing buffer (50% 100 mM ammonium bicarbonate/50% acetonitrile) and washed twice for 15 min each with vortexing. Gel pieces were washed and dried in 100% acetonitrile (ACN) followed by drying in a SpeedVac. Dried gel pieces were re-swollen in a 12.5 ng/µL trypsin (Promega, Germany) solution reconstituted with 25 mM ammonium bicarbonate or 12.5 ng/µL chymotrypsin (Roche, Germany) solution buffered in 25 mM ammonium bicarbonate. Gel pieces were incubated for 16 h (overnight) at 37°C (trypsin) or 25°C (chymotrypsin). The supernatant was transferred to new 0.5 mL tubes, and peptides were extracted with 50 µL of 0.5% formic acid / 20% acetonitrile for 20 min in a sonication bath. This step was repeated two times. Samples in extraction buffer were pooled in 0.5 mL tubes and evaporated in a SpeedVac concentrator. The volume was reduced to approximately 20 µL and then 20 µL HPLC grade water (Sigma, Germany) was added (Ghafari et al., Journal of proteome research 2012 Mar 2; 11(3): 1891-1896).

### Mass spectrometry

Samples were analysed by injecting 15 µl into a Dionex 3000 nano-LC system equipped with C18 Acclaim Pepmap100 columns (all Thermo Fisher Scientific, Austria). Samples were loaded on the precolumn for 10 min at a flow rate of 10 µl/min using 100% mobile phase A and separated at a flow rate of 300 nl/min using a linear gradient of 45 min from 7% to 40% mobile phase B starting 1 min after valve switching and followed by 10 min equilibration after the gradient with a total run time of 66 min (mobile phase A: 2% ACN, 0.2% formic acid, mobile phase B: 80% ACN, 0.2% formic acid; all solvents LC-MS grade). Analytes were ionised using a nanospray ion source (Thermo Fisher Scientific, Austria) and MS analysis was performed on a QExactive Orbitrap (Thermo Fisher Scientific, Austria). Peptides were detected over a scan range from 400 to 1400 Th with a target mass resolution of R= 70000 at m/z 200. The instrument was operated in DDA mode with MS/MS scans of the top 6 precursor ions from the preceding MS scan in the HCD trap of the instrument (normalized collision energy set to 30%, resolution set to 17500) (Groessl et al., Proteomics 2012 Sep; 12(18): 2833-2842).

MASCOT searches were done by using the MASCOT 2.2.06 (Matrix Science, London, U.K.) against latest UniProtKB database for protein identification. Searching parameters were set as follows: enzyme selected as trypsin or chymotrypsin with three and five maximum missing cleavage sites respectively, species taxonomy: limited to human, a mass tolerance of 5 ppm for peptide tolerance, 20 mmu for MS/MS tolerance, ions score cutoff lower than 15, fixed modification of carbamidomethyl (C) and variable modification of oxidation (M), deamidation (N, Q), and phosphorylation (S, T, Y). Positive protein identifications were based on a significant MOWSE score. After protein identification, an error-tolerant search was done to detect unspecific cleavage and unassigned modifications. Protein identification information returned were manually inspected and filtered to obtain confirmed protein identification lists.

Higher sequence coverages were obtained using Modiro^{®} software with following parameters: enzyme selected as used with three and five maximum missing cleavage sites, a peptide mass tolerance of 0.2 Da for peptide tolerance, 0.2 Da for fragment mass tolerance, modification 1 of carbamidomethyl(C) and modification 2 of methionine oxidation. Positive protein identification was first of all listed by spectra view and subsequently each identified peptide was considered significant based on the ioncharge status of peptide, b- and y-ion fragmentation quality, ion score (>200) and significance scores (>80). Protein identification were manually inspected and filtered to obtain confirmed protein identification lists (Ghafari et al., Journal of proteome research 2012 Mar 2; 11(3): 1891-1896).

### Statistical evaluation

Data from Western blotting were handled by Student's t test and data are given as means ± SD. Calculations as well as Pearson correlations between data were carried out using SPSS for windows 19.0.

To show whether receptor complexes contained individual subunits a comparative blot was constructed.

### Results

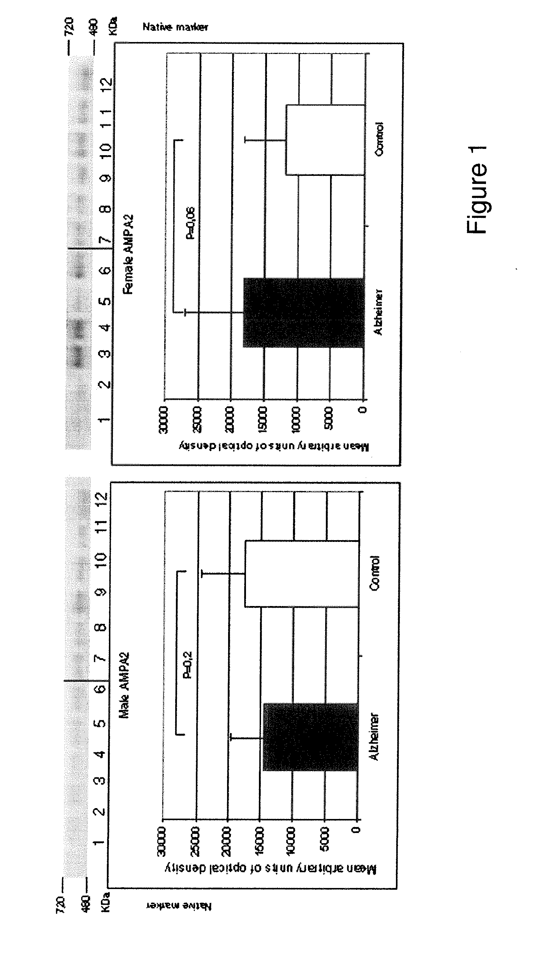

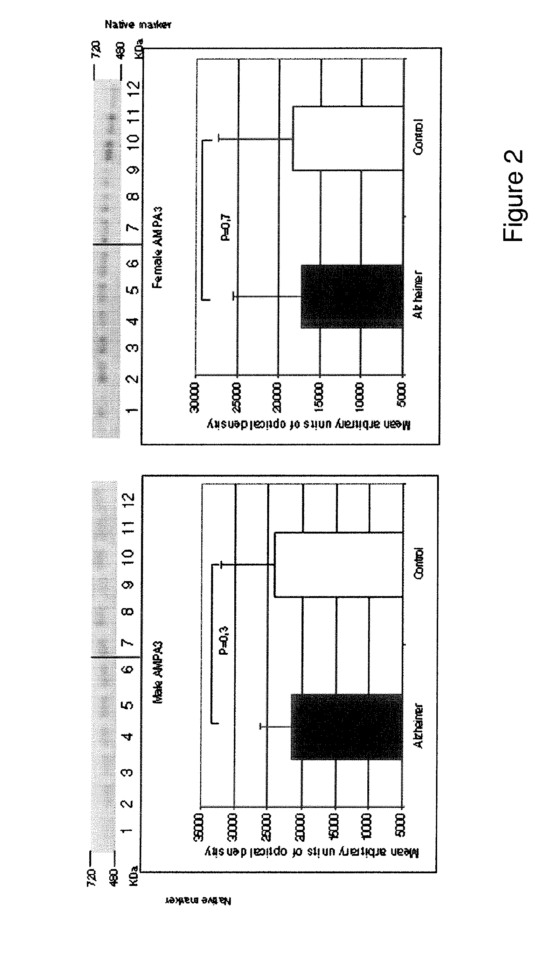

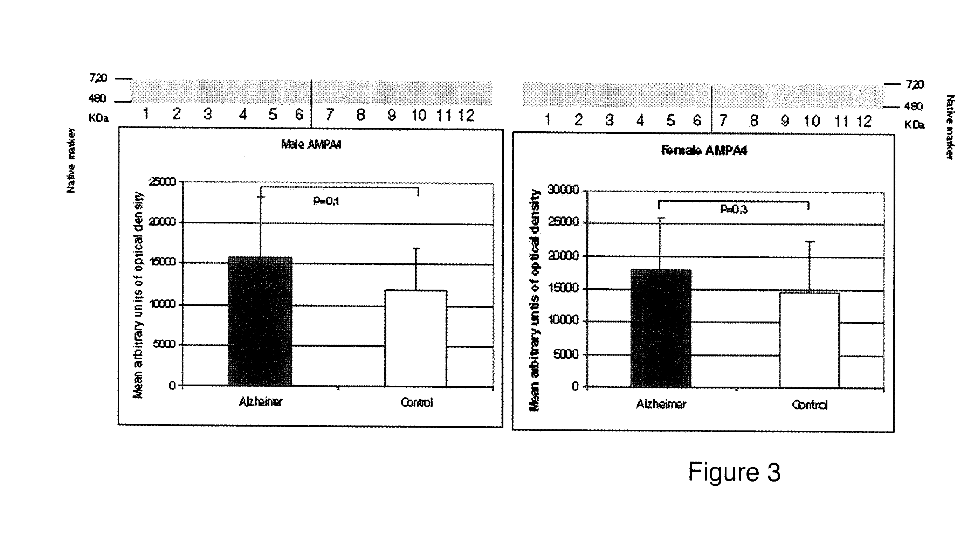

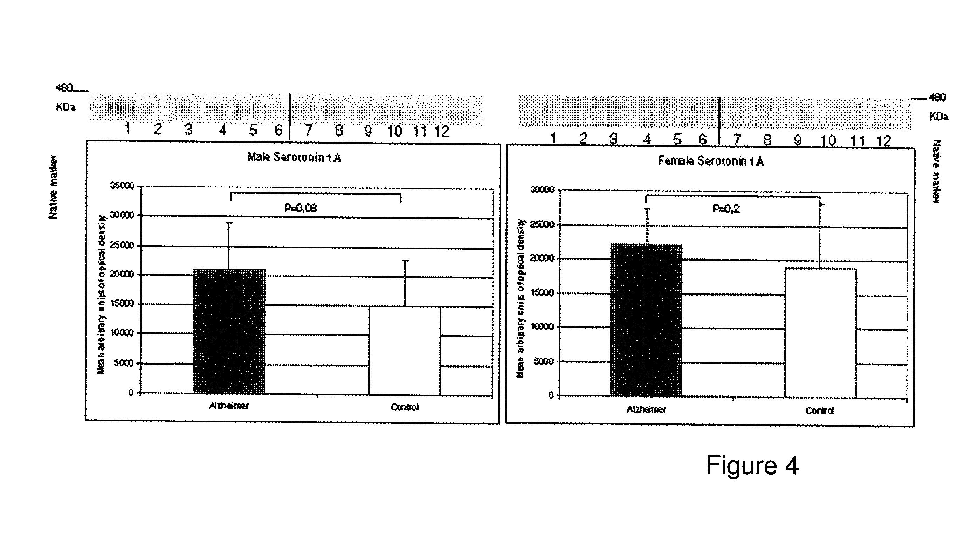

Receptor complex levels were comparable for complexes containing GluR2, GluR3 and GluR4 as well as 5-HT1A and the results are demonstrated in figures 1-4. Also mobilities of complexes were comparable. This finding may indicate that these receptor complexes containing the subunits GluR2-4 and 5-HT1A are qualitatively and quantitatively unchanged.

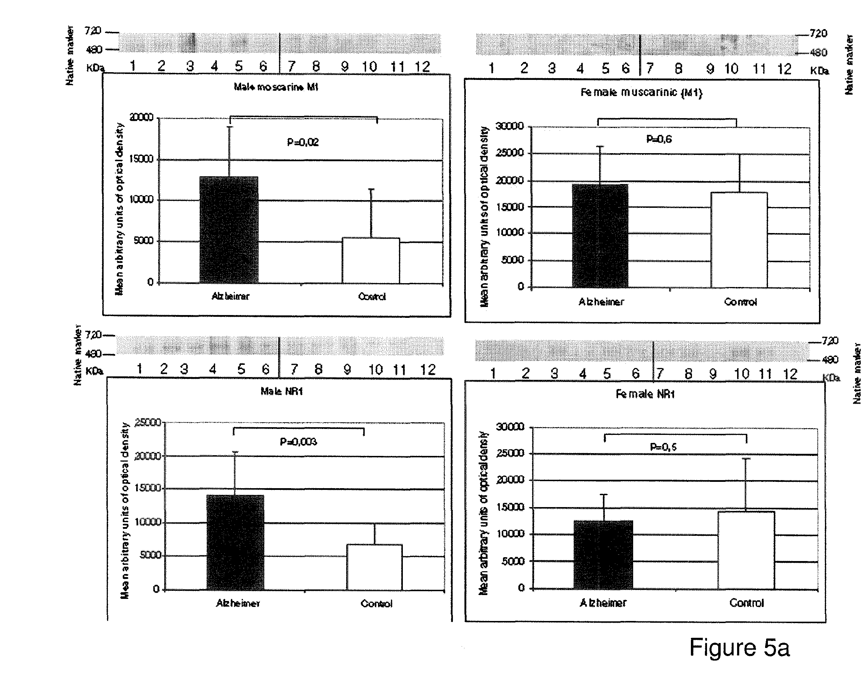

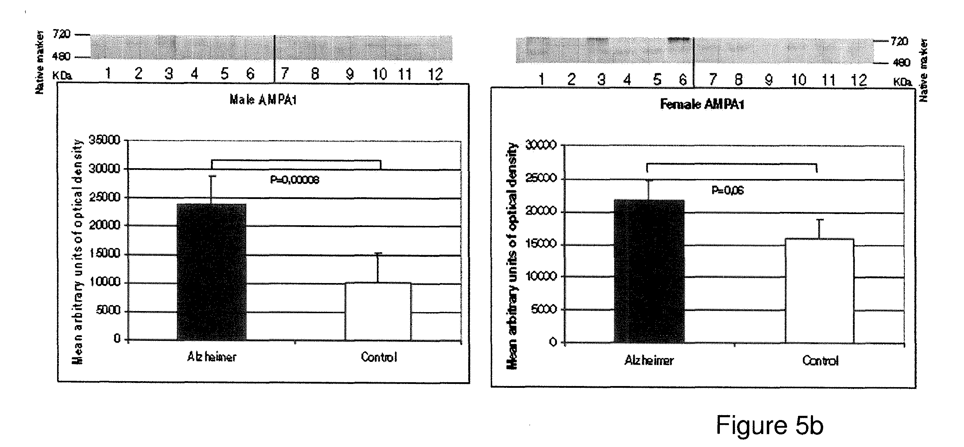

Receptor complex levels containing muscarinic AChR M1, NR1 and GluR1 were significantly increased in male patients with AD (figure 5). No changes in electrophoretic mobility were observed in either group.

A single band was observed between 480 and 720kDa for M1, between 480 and 720kDa but with lower mobility than M1 for NR1 and between 480 and 720 kDa with the same mobility as GluR2-4 and different from M1 and NR1.

These three receptor complex levels were significantly increased in male patients with AD as compared to control individuals. The electrophoretic pattern of mobility was comparable between groups.

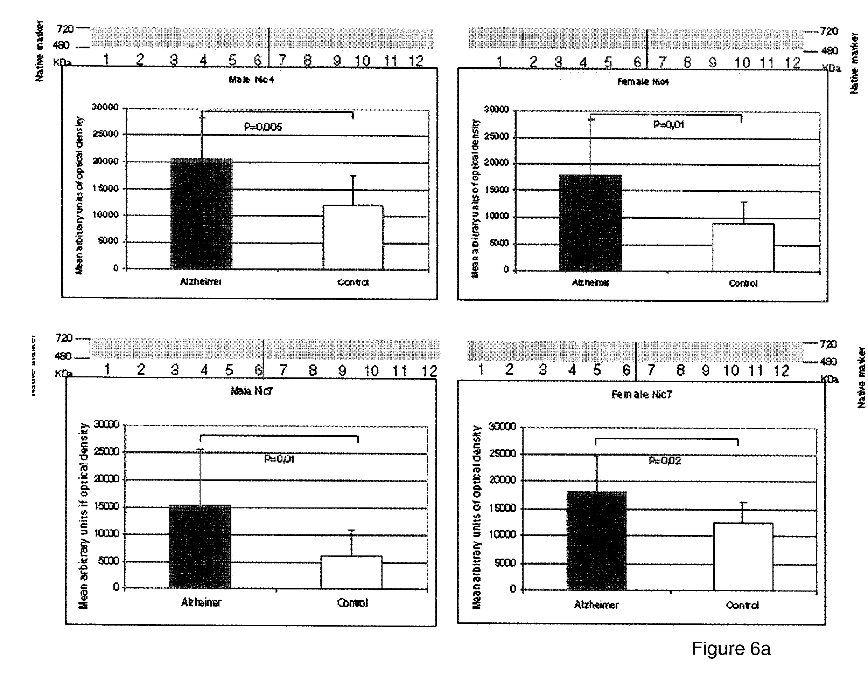

As shown in figure 6a Nicotinic AChRs 4 and 7 receptor complex levels were increased in both sexes. Both nAChR4 and 7 containing receptor complex levels were comigrating at the same position between 480 and 720 kDa. Electrophoretic mobility was comparable between groups.

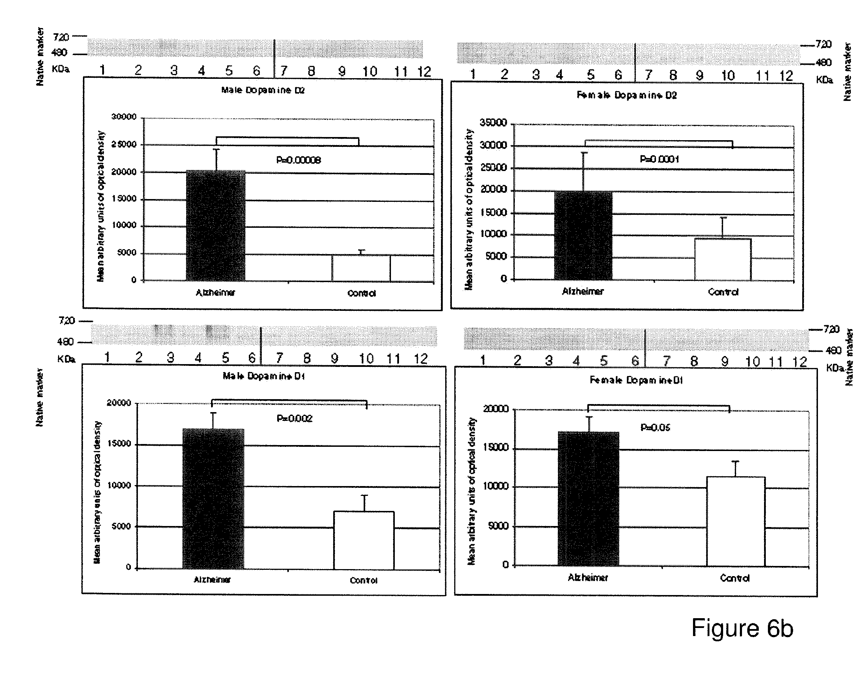

Receptor complexes containing dopamine receptors D1 and D2 were increased in males and females with AD and were migrating between 480 and 720 kDa showing different mobility (Figure 6b). Both receptor complexes, however, showed comparable electrophoretic mobility in controls and patients with AD.

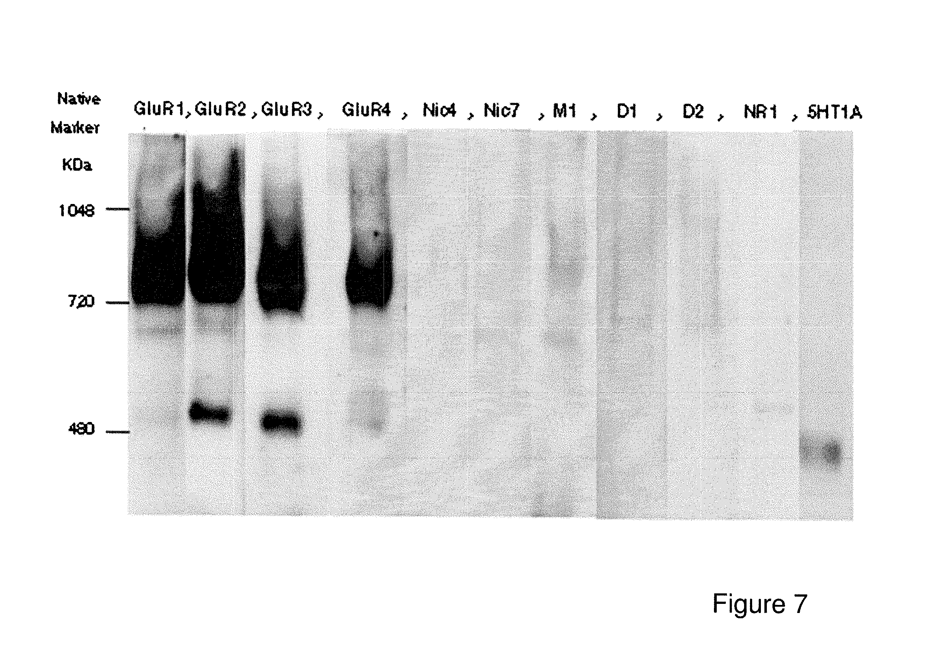

A blot comparing mobility of receptor complexes containing the individual subunits is shown in figure 7.

The receptor complex containing AChR subunit M1 showed mobility that was different from all other receptor complexes observed herein but showed comparable mobility to nAChR 4 and 7. NR1 containing receptor complexes migrated at a position different from migrations of nAChR 4 and 7. All AMPA receptor subunits (GluR1-4) containing complexes were observed in an identical electrophoretic position that was different from other receptor complexes. D1 and D2 containing complexes showed comparable mobilities between each other and different from all other receptor complexes. The 5HT1A containing receptor complex showed individual and different mobility that was not comparable to other receptor complexes.

A series of receptor complexes were correlating in controls as well as in patients with AD. The correlation patterns, however, were different between controls and patients with AD, probably indicating deterioration of the concerted action of brain receptor complexes.

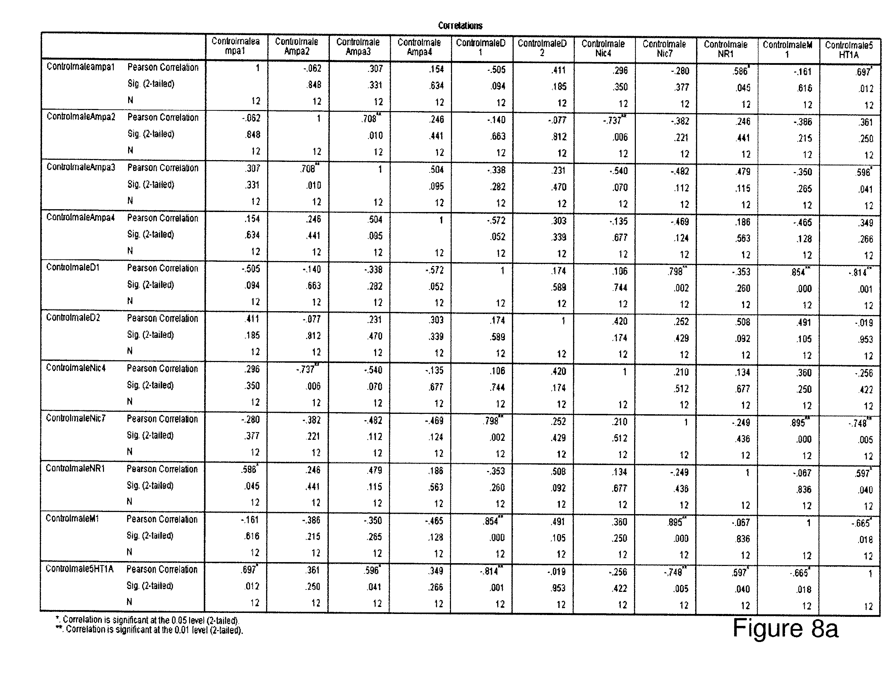

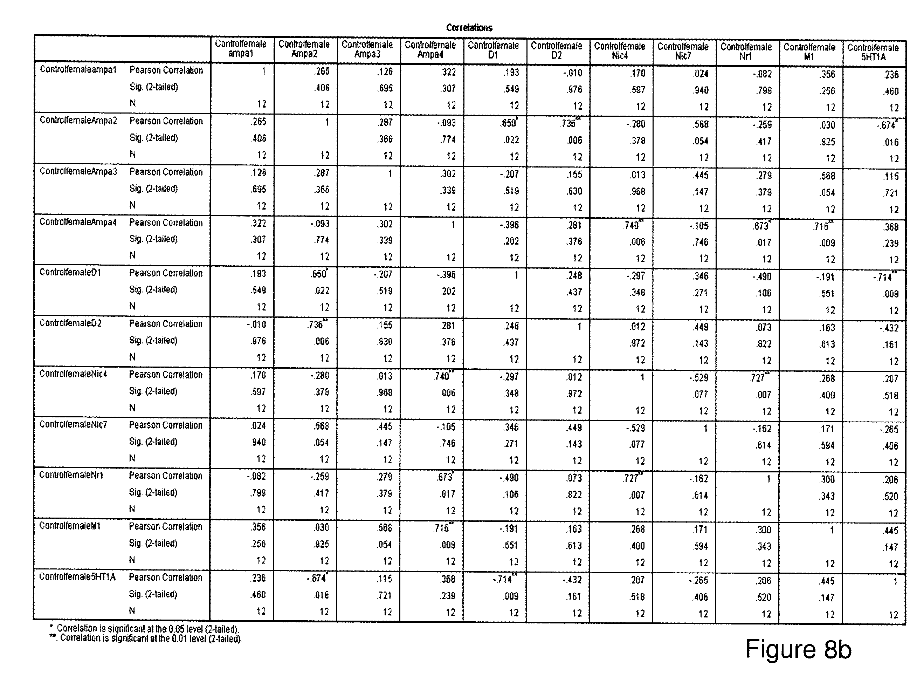

Correlation between receptor complexes in males and females from the control group (table 2; Figure 8a, b).

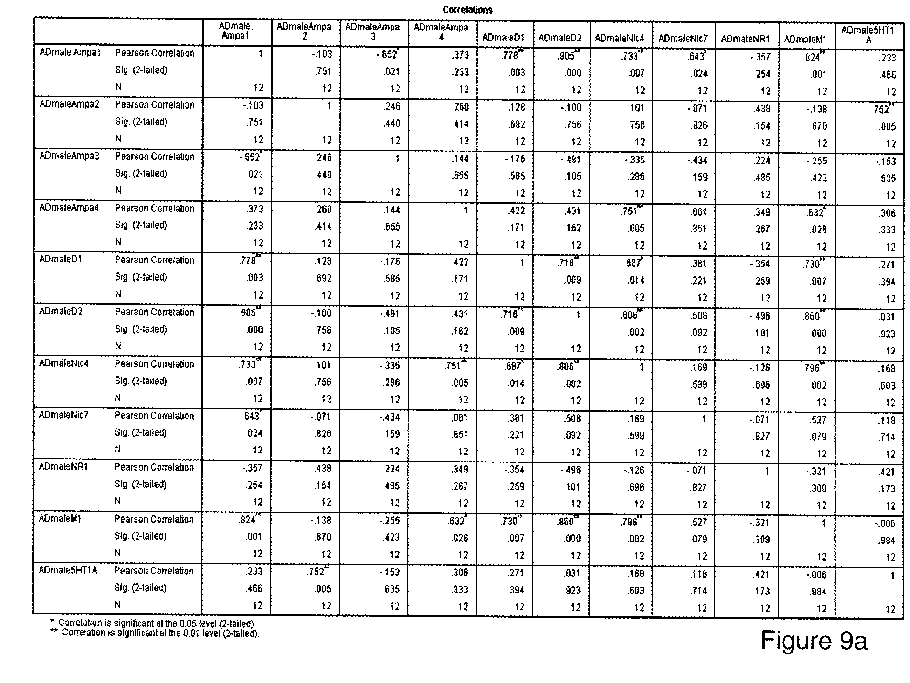

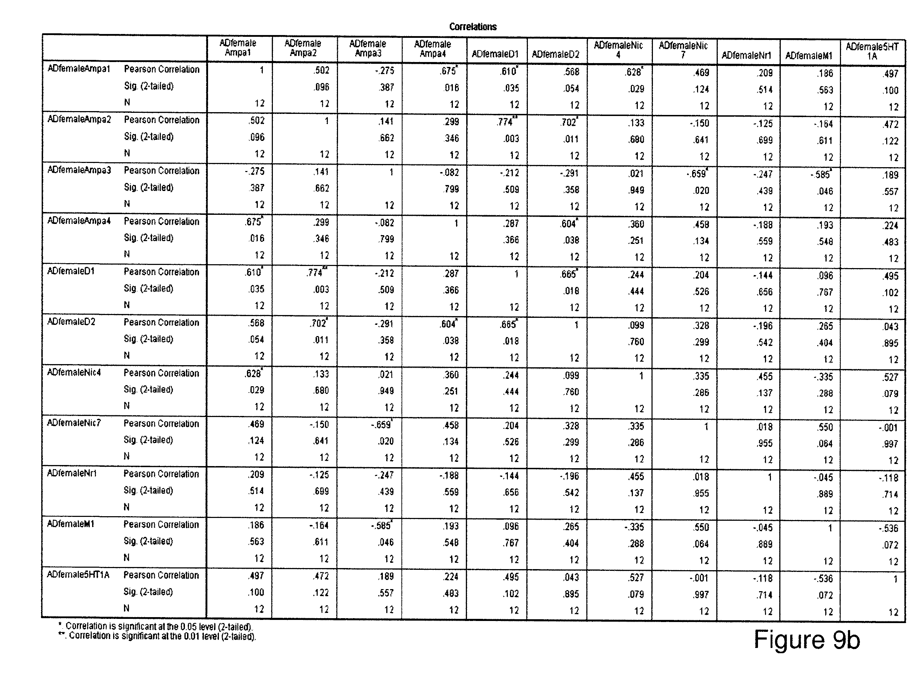

Correlation between receptor complexes in males and females from the Alzheimer group (table 3; Figure 9a, b).

**Table 2:**

| Male Control | Correlation | | | | | |
|---|---|---|---|---|---|---|
| Receptor Complexes | GluR3 | nAChR4 | nAChR7 | NR1 | M1 | 5-HT1_{A} |
| GluR1 | | | | 0.04* | | 0.012* |
| GluR2 | 0.010** | 0.006** | | | | |
| GluR3 | | | | | | 0.04* |
| D1 | | | 0.002** | | 0.000** | 0.001** |
| nAChR7 | | | | | 0.000** | 0.005** |
| NR1 | | | | | | 0.04* |
| M1 | | | 0.000** | | | 0.01* |

| Female Control | Correlation | | | | | |
|---|---|---|---|---|---|---|
| Receptor Complexes | D1 | D2 | nAChR4 | nAChR7 | NR1 | M1 |
| GluR2 | 0.02* | 0.006** | | 0.05* | | |
| GluR4 | | | 0.006** | | 0.01* | 0.009** |
| nAChR4 | | | | | 0.007** | |

**Table 3**

| Male Alzheimer | Correlation | | | | | | | |
|---|---|---|---|---|---|---|---|---|
| Receptor Complexes | GluR1 | GluR3 | D1 | D2 | nAChR4 | nAChR7 | M1 | 5-HT1_{A} |
| GluR1 | | 0.02* | 0.003** | 0.000** | 0.007** | 0.02* | 0.001** | |
| GluR2 | | | | | | | | 0.005* * |
| GluR3 | 0.02* | | | | | | | |
| GluR4 | | | | | 0.005** | | 0.02* | |
| D1 | 0.003 ** | | | 0.009** | 0.01* | | 0.007** | |
| D2 | 0.000 ** | | 0.009** | | 0.002** | | 0.000** | |
| nAChR4 | 0.007 ** | | 0.01* | 0.002** | | | 0.002** | |

| Female Alzheimer's | Correlation | | | | | | | |
|---|---|---|---|---|---|---|---|---|
| Receptor Complexes | GluR1 | GluR4 | D1 | D2 | nAChR4 | nAChR7 | M1 | |
| GluR1 | | 0.01* | 0.03 | | 0.02* | | | |
| GluR2 | | | 0.003** | 0.01* | | | | |
| GluR3 | | | | | | 0.02* | 0.04* | |
| GluR4 | 0.01* | | | 0.03* | | | | |
| D1 | 0.03* | | | 0.01* | | | | |
| D2 | | 0.03* | 0.01* | | | | | |

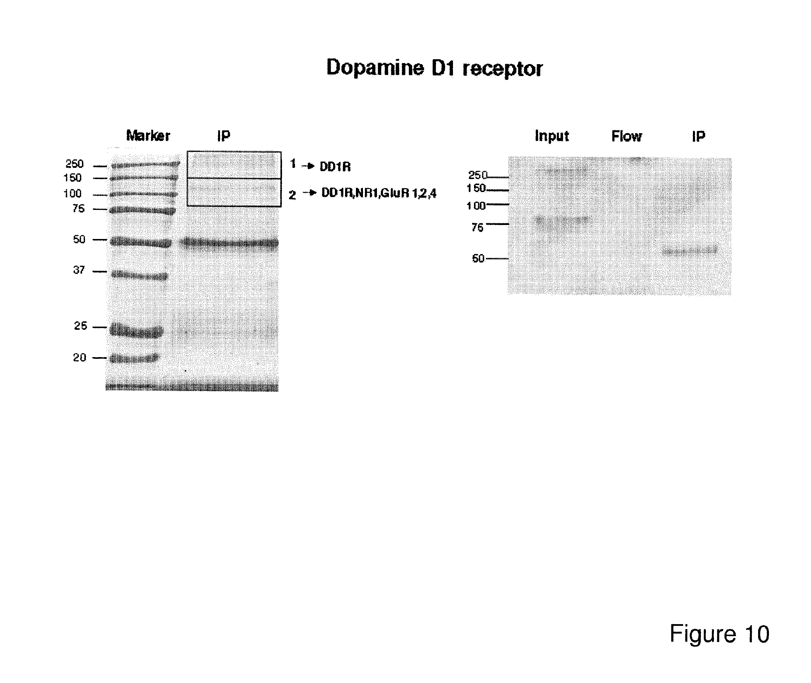

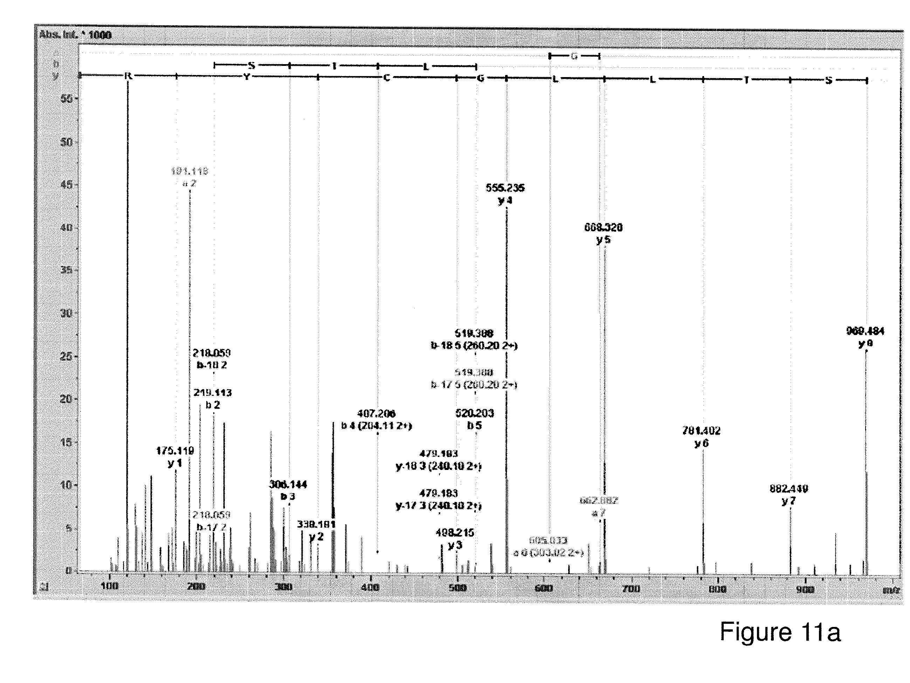

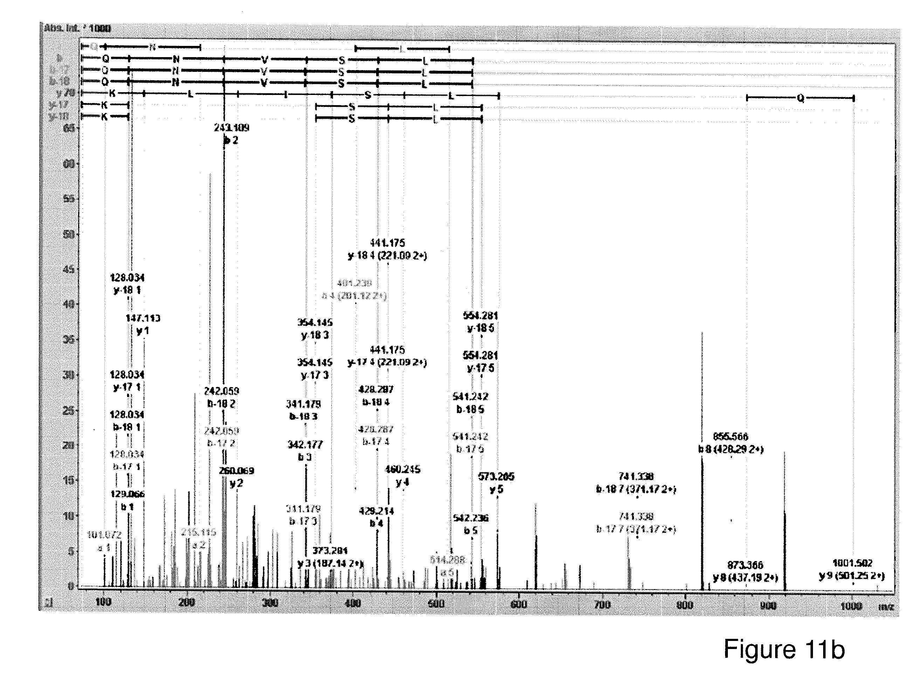

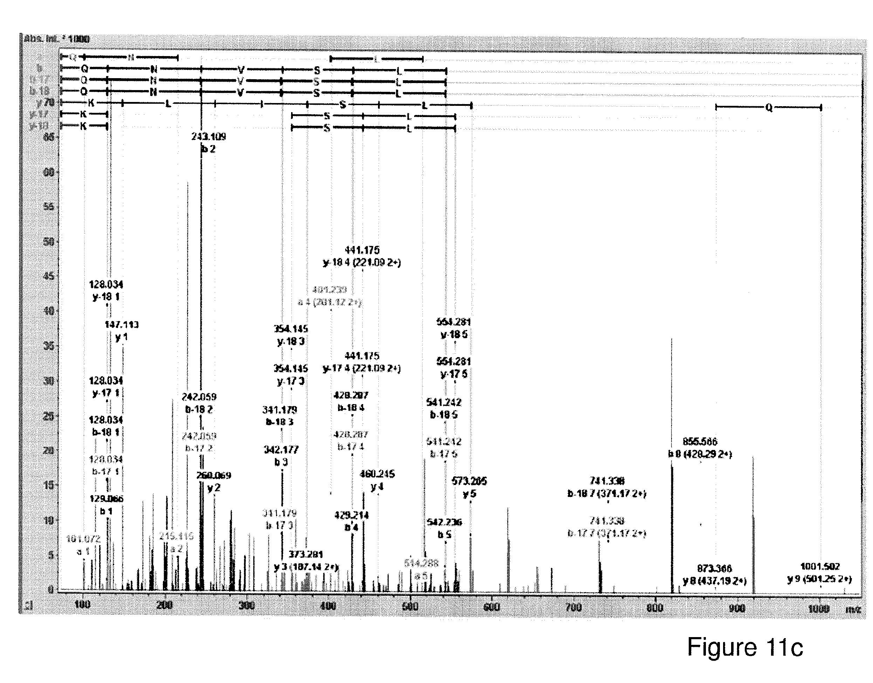

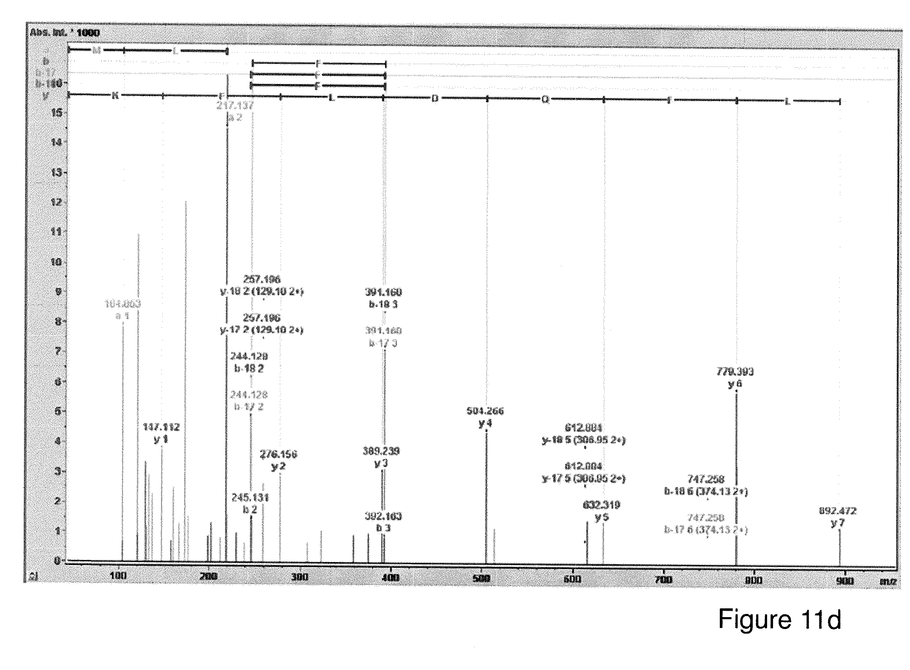

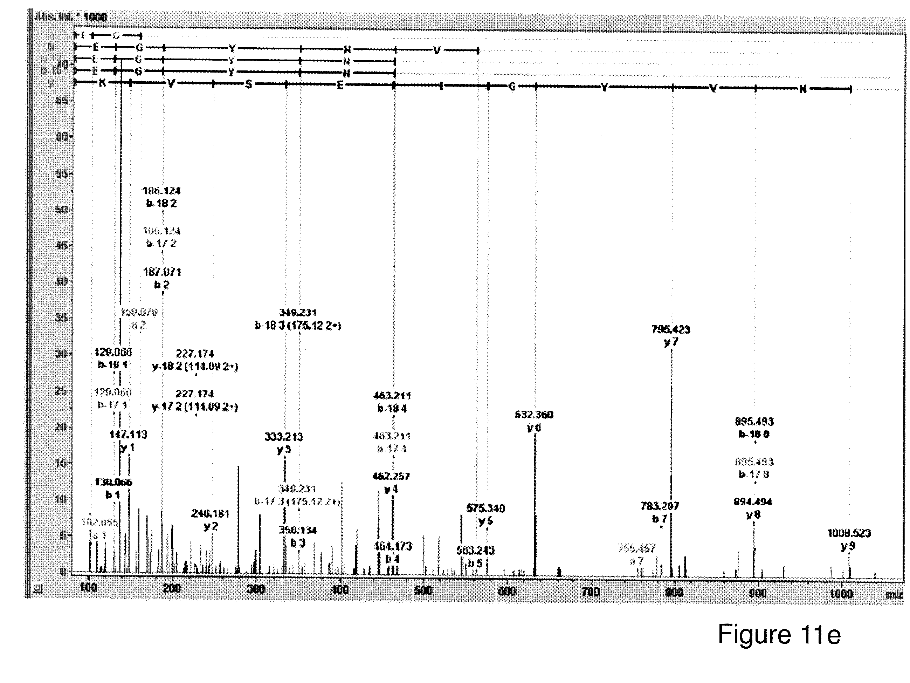

Binding partners indicating the presence of several receptor subtypes in the D1 receptor complexes are given in figure 10 as revealed by immunoprecipitation followed by mass spectrometrical identification. Representative mass spectrometrical spectra are shown in figure 11a to 11e.

The list of peptides identified are given in table 4.

**Table 4:**

| P21728 DRD1_HUMAN Dopamine D1 R | | | | | | | |
|---|---|---|---|---|---|---|---|
| R | enzyme | Start - End | Observed | Mr(expt) | error(Da) | Sequence | score |
| D(1A) dopamine R | Try | 122 - 130 | 563,787 | 1125,5594 | -0,0013 | R.YWAISSPFR.Y | 21 |
| D(1A) dopamine R | Try | 340 - 349 | 594,2974 | 1186,5803 | -0,0002 | K.AFSTLLGCYR.L | 29 |
| D(1A) dopamine R | Try | 384 - 403 | 568,7971 | 2271,1594 | 0,0025 | K.ECNLVYLIPHAVGSSEDLKK.E | 15 |
| D(1A) dopamine R | Try | 122 - 130 | 563,7872 | 1125,5598 | -0,0009 | R.YWAISSPFR.Y | 38 |
| D(1A) dopamine R | Try | 122 - 130 | 563,7872 | 1125,5598 | -0,0009 | R.YWAISSPFR.Y | 25 |
| D(1A) dopamine R | Try | 340 - 349 | 594,2976 | 1186,5805 | 0,0001 | K.AFSTLLGCYR.L | 38 |
| D(1A) dopamine R | Try | 340 - 349 | 594,2976 | 1186,5805 | 0,0001 | K.AFSTLLGCYR.L | 28 |
| D(1A) dopamine R | Try | 340 - 349 | 594,2978 | 1186,581 | 0,0006 | K.AFSTLLGCYR.L | 24 |
| D(1A) dopamine R | Try | 340 - 349 | 594,2979 | 1186,5811 | 0,0007 | K.AFSTLLGCYR.L | 28 |
| D(1A) dopamine R | Ctr | 390 - 413 | 626,098 | 2500,3627 | -0,0021 | Y.LIPHAVGSSEDLKKEEAAGIARPL.E | 58 |
| D(1A) dopamine R | Try | 339 - 349 | 594,2974 | 594,345 | -0,0476 | R.KAFSTLLGCYR.L | 257/99.7 |
| D(1A) dopamine R | Try | 350 - 378 | 1011,795 | 1011,8189 | -0,0231 | R.LCPATNNAIETVSINNNGAAMFSSHHEPR.G | 225/80 |
| D(1A) dopamine R | Try | 134 - 148 | 554,6171 | 554, 6639 | -0,0468 | R.KMTPKAAFILISVAW.T | 332/80 |
| D(1A) dopamine R | Ctr | 154 - 163 | 643,2958 | 642,8344 | 0,4614 | L.ISFIPVQLSW.H | 208/95.3 |
| D(1A) dopamine R | Ctr | 52 - 62 | 682,341 | 682,3966 | -0,0556 | F.RHLRSKVTNFF.V | 206/95.3 |
| D(1A) dopamine R | Ctr | 390 - 413 | 626,098 | 626,0985 | -0,0006 | Y.LIPHAVGSSEDLKKEEAAGIARPL.E | 436/94.8 |
| D(1A) dopamine R | Ctr | 275 - 285 | 646,317 | 646,798 | -0,481 | L.SVIMGVFVCCW.L | 310/90 |
| D(1A) dopamine R | Ctr | 265 - 274 | 590,8687 | 590,8928 | -0,0242 | F.KRETKVLKTL.S | 215/90 |
| D(1A) dopamine R | Ctr | 115 - 123 | 405,2389 | 405,1884 | 0,0505 | L.CVISVDRYW.A | 300/80.7 |
| D(1A) dopamine R | Ctr | 103 - 114 | 614,3402 | 614,8231 | -0,4829 | F.DIMCSTASILNL.C | 206/80 |
| | | | | | | | |
| | | | | | | | |
| | | | | | | | |

| Q05586 NMDZ1_HUMAN Glu [NMDA] R SU zeta-1 | | | | | | | |
|---|---|---|---|---|---|---|---|
| R | enzyme | Start - End | Observed | Mr(expt) | error(Da) | Sequence | score |
| Glu [NMDA] R SU zeta-1 | Try | 26 - 36 | 571,8484 | 1141,6822 | 0,0003 | K.IVNIGAVLSTR.K | 40 |
| Glu [NMDA] R SU zeta-1 | Try | 26 - 36 | 571,8484 | 571,8482 | 0,0002 | K.IVNIGAVLSTR.K | 424/98.2 |
| Glu [NMDA] R SU zeta-1 | Try | 696 - 704 | 627,857 | 627,7817 | 0,0753 | R.QVELSTMYR.H | 256/97.8 |
| Glu [NMDA] R SU zeta-1 | Try | 770 - 778 | 597,3058 | 597,3031 | 0,0027 | K.QNVSLSILK.S | 210/96.2 |
| Glu [NMDA] R SU zeta-1 | Try | 770 - 778 | 609,8289 | 609,8031 | 0,0258 | K.QNVSLSILK.S | 217/95.8 |
| Glu [NMDA] R SU zeta-1 | Try | 433 - 448 | 870,9675 | 870,9174 | 0,0501 | K.KVICTGPNDTSPGSPR.H | 200/91 |
| Glu [NMDA] R SU zeta-1 | Try | 532 - 544 | 756,853 | 756,9714 | -0,1184 | K.PFKYQGLTILVKK.E | 206/86.1 |
| Glu [NMDA] R SU | Try | 914 - 924 | 651,7921 | 651,886 | -0,0939 | K.DTVLPRRAIER.E | 200/80.1 |
| zeta-1 | | | | | | | |
| Glu [NMDA] R SU zeta-1 | Try | 907 - 919 | 759,4143 | 759,4019 | 0,0124 | R.GALQNQR.R | 242/80 |
| Glu [NMDA] R SU zeta-1 | Try | 422 - 432 | 633,7947 | 633,7961 | -0,0013 | K.EEFTVNGDPVK.K | 200/96 |
| Glu [NMDA] R SU zeta-1 | Try | 899 - 913 | 700,8901 | 701,3713 | -0,4812 | K.DTSTGGGRGALQNQK.D | 230/80 |
| Glu [NMDA] R SU zeta-1 | Try | 920 - 934 | 892, 9132 | 892,9627 | -0,0495 | R.RAIEREEGQLQLCSR.H | 237/80 |
| | | | | | | | |
| | | | | | | | |
| | | | | | | | |

| P42261 GRIA1_HUMAN Glu R 1 | | | | | | | |
|---|---|---|---|---|---|---|---|
| R | enzyme | Start - End | Observed | Mr(expt) | error(Da) | Sequence | score |
| Glu R 1 | Try | 209 - 218 | 541,8506 | 1081,6866 | 0,0007 | R.LNAILGQIIK.L | 29 |
| Glu R 1 | Try | 656 - 670 | 784,894 | 1567,7735 | 0,0006 | K.QTEIAYGTLEAGSTK.E | 38 |
| Glu R 1 | Try | 44 - 54 | 615,8399 | 1229,6652 | -0,0003 | R.FALSQLTEPPK.L | 17 |
| Glu R 1 | Try | 187-194 | 512,2619 | 1022,5092 | -0,0014 | R.MLFQDLEK.K | 47 |
| Glu R 1 | Try | 209 - 218 | 541,8505 | 1081,6865 | 0,0006 | R.LNAILGQIIK.L | 29 |
| Glu R 1 | Try | 656 - 670 | 784,8944 | 1567,7743 | 0,0013 | K.QTEIAYGTLEAGSTK.E | 44 |
| Glu R 1 | Try | 862 - 877 | 732,8227 | 732,7929 | 0,0298 | R.NSGAGASSGGSGENGR.V | 200/99.7 |
| Glu R 1 | Try | 209 - 218 | 541,8506 | 541,8502 | 0,0003 | R.LNAILGQIIK.L | 447/91.3 |
| Glu R 1 | Try | 362 - 372 | 632,304 | 632,3501 | -0,0461 | R.TNYTLHVIEMK.H | 288/89 |
| Glu R 1 | Try | 731 - 744 | 858,8912 | 858,8422 | 0,049 | K.PCDTMKVGGNLDSK.G | 200/84 |
| Glu R 1 | Try | 435 - 448 | 801,379 | 801,3794 | -0,0004 | R.YEGYCVELAAEIAK.H | 229/99.1 |
| Glu R 1 | Try | 758 - 766 | 455,7897 | 455,8004 | -0,0107 | R.NPVNLAVLK.L | 331/90.5 |
| Glu R 1 | Try | 55 - 73 | 1120,567 | 1120,567 | 0 | K.LLPQIDIVNISDSFEMTYR.F | 220/89.2 |
| Glu R 1 | Try | 199 - 208 | 617,8208 | 617,7952 | 0,0256 | R.LVVVDCESER.L | 255/88.5 |
| Glu R 1 | Try | 737 - 752 | 766,8724 | 766,9201 | -0,0477 | K.VGGNLDSKGYGIATPK.G | 238/86.5 |
| Glu R 1 | Try | 767 - 779 | 732,8488 | 732,9408 | -0,0921 | K.LNEQGLLDKLKNK.W | 255/84.9 |
| Glu R 1 | Try | 81 - 91 | 640,3217 | 640,3324 | -0,0107 | K.GVYAIFGFYER.R | 345/99.7 |
| | | | | | | | |
| | | | | | | | |
| | | | | | | | |

| P42262 GRIA2_HUMAN Glu R 2 | | | | | | | |
|---|---|---|---|---|---|---|---|
| R | enzyme | Start - End | Observed | Mr (expt) | error(Da) | Sequence | score |
| Glu R 2 | Try | 25 - 35 | 601,3376 | 1200,6606 | -0,0009 | S.NSIQIGGLFPR.G | 42 |
| Glu R 2 | Try | 84 - 94 | 640,3217 | 1278,6289 | 0,0004 | R.GVYAIFGFYDK.K | 44 |
| Glu R 2 | Try | 386 - 394 | 548,7694 | 1095,5241 | 0,0005 | K.IGYWSEVDK.M | 37 |
| Glu R 2 | Try | 493 - 506 | 733,9498 | 1465,8851 | -0,0017 | K.ADIAIAPLTITLVR.E | 42 |
| Glu R 2 | Try | 663 - 677 | 785,8825 | 1569,7505 | -0,0018 | K.QTEIAYGTLDSGSTK.E | 70 |
| Glu R 2 | Try | 851 - 870 | 738,3447 | 2212,0124 | -0,0021 | K.NAQNINPSSSQNSQNFATYK.E | 53 |
| Glu R 2 | Try | 871 - 882 | 679,3346 | 1356,6546 | -0,0015 | K.EGYNVYGIESVK.I | 34 |
| Glu R 2 | Try | 232 - 249 | 670,6818 | 2009,0236 | -0,0022 | K.GYHYIIANLGFTDGDLLK.I | 45 |
| Glu R 2 | Try | 442 - 455 | 801,379 | 1600,7435 | -0,0008 | R.YEGYCVDLAAEIAK.H | 39 |
| Glu R 2 | Try | 493 - 506 | 733,9501 | 1465,8857 | -0,0011 | K.ADIAIAPLTITLVR.E | 52 |
| Glu R 2 | Try | 663 - 677 | 785,8833 | 1569,752 | -0,0002 | K.QTEIAYGTLDSGSTK.E | 73 |
| Glu R 2 | Try | 851 - 870 | 738,3455 | 2212,0147 | 0,0003 | K.NAQNINPSSSQNSQNFATYK.E | 44 |
| Glu R 2 | Ctr | 112 - 124 | 708,8432 | 1415,6718 | -0,0003 | F.ITPSFPTDGTHPF.V | 33 |
| Glu R 2 | Try | 663 - 677 | 785,8825 | 785,8834 | -0,0009 | K.QTEIAYGTLDSGSTK.E | 337/100 |
| Glu R 2 | Try | 871 - 882 | 679,3346 | 679,3353 | -0,0008 | K.EGYNVYGIESVK.I | 270/99.9 |
| Glu R 2 | Try | 851 - 870 | 738,3447 | 738,3454 | -0,0007 | K.NAQNINPSSSQNSQNFATYK.E | 337/99.9 |
| Glu R 2 | Try | 25 - 35 | 601,3376 | 601,338 | -0,0009 | S.NSIQIGGLFPR.G | 392/99.8 |
| Glu R 2 | Try | 993 - 506 | 733,9498 | 733,9507 | -0,0009 | K.ADIAIAPLTITLVR.E | 344/99.8 |
| Glu R 2 | Try | 738 - 751 | 858,8912 | 858,8422 | 0,049 | K.PCDTM^{OK}KVGGNL^{236.1}DSK.G | 200/84 |
| Glu R 2 | Try | 194 - 202 | 503,2494 | 503,3004 | -0,051 | R.SLFQDLEL^{-87.09}K.K | 321/83.1 |
| Glu R 2 | Ctr | 809 - 818 | 417,9205 | 417,8502 | 0,0703 | L.SLSNVA^{195.21}GVFY.I | 433/100 |
| Glu R 2 | Ctr | 548 - 554 | 606,3085 | 606,1981 | 0,1104 | W.M^{365.22}CIVFAY.I | 239/99.6 |
| Glu R 2 | Ctr | 159 - 173 | 793,3859 | 793,441 | -0,0551 | L.STLQAVLDSAAEK^{-61.1}KW.Q | 214/98.9 |
| Glu R 2 | Ctr | 601 - 617 | 673,3236 | 673,2923 | 0,0313 | F.SLGAFMQQGCDISP^{208.09}RSL.S | 306/90.9 |
| Glu R 2 | Ctr | 460 - 471 | 664,8814 | 664,8954 | -0,014 | F.KYKLTIVGDG^{-56.02}KY.G | 223/90.1 |
| Glu R 2 | Ctr | 261 - 273 | 799,906 | 799,8801 | 0,0259 | F.QIVDYDD^{10.05}SLVSKF.I | 203/89.4 |
| Glu R 2 | Ctr | 645 - 660 | 569,3048 | 569,3064 | -0,0017 | F.LTVERM^{-83.}VSPIESAEDL.S | 229/81.8 |
| | | | | | | | |
| | | | | | | | |
| | | | | | | | |

| P48058 GRIA4_HUMAN Glu R 4 | | | | | | | |
|---|---|---|---|---|---|---|---|
| R | enzyme | Start - End | Observed | Mr(expt) | error(Da) | Sequence | score |
| Glu R 4 | Try | 669 - 678 | 785,8825 | 1569,7505 | -0,0018 | K.QTEIAYGTLDSGSTK.E | 70 |
| Glu R 4 | Try | 766 - 774 | 477,8022 | 953,5899 | -0,0011 | R.TPVNLAVLK.L | 38 |
| Glu R 4 | Try | 664 - 678 | 785,8833 | 1569,752 | -0,0002 | K.QTEIAYGTLDSGSTK.E | 73 |
| Glu R 4 | Try | 761 - 774 | 678,3982 | 1354,7818 | -0,0002 | K.GSSLRTPVNLAVLK.L *Arg→Gly (R)* | 30 |
| Glu R 4 | Try | 766 - 774 | 477,8029 | 953,5912 | 0,0002 | R.TPVNLAVLK.L | 38 |
| Glu R 4 | Try | 664 - 678 | 785,8825 | 785,8834 | -0,0009 | K.QTEIAYGTLDSGSTK.E | 337/100 |
| Glu R 4 | Try | 494 - 507 | 733,9498 | 733,9507 | -0,0009 | K.A^{-G-}EIAIAPLTITLVR.E | 327/99.5 |
| Glu R 4 | Try | 846 - 856 | 641,8213 | 641,8768 | -0,0555 | K.RM^{Ox}KLTFSERI^{-85.1}R.N | 208/96.2 |
| Glu R 4 | Try | 739 - 752 | 858,8912 | 858,8422 | 0,049 | K. PCDTM^{Ox}KVGGNL^{236.1}DSK.G | 200/84 |
| Glu R 4 | Try | 279 - 285 | 601,3376 | 601,2825 | 0,0551 | R.WKKLD^{228.11}QR.E | 279/81.5 |
| Glu R 4 | Try | 761 - 785 | 663,3033 | 663,4021 | -0,0988 | K.GS^{Me}SLRTPVNLAVLKLS^{Me}EAGVLDK^{Me}LK.N | 224/80 |
| Glu R 4 | Try | 443 - 456 | 801,379 | 801,3794 | -0,0004 | K.YEGYC^{CAMe}VDLAS^{-A-}EIAK.H | 229/99.1 |

Information on sequence coverage is provided in table 5.

**Table 5**

| accession number | enzyme | sequence coverage | molecular weight (expected) -kDa | total amino acids | total sequence coverage |
|---|---|---|---|---|---|
| P21728 | Trypsin | 19% | 49.293 | 446 | 35% |
| Dopamine D1 receptor | Chymotrypsin | 20% | | | |
| P42261 GluR1 | Trypsin | 18% | 101.506 | 906 | 18% |
| P42262 GluR-2 | Trypsin | 17% | 98.821 | 883 | 28% |
| P48058 GluR-4 | Trypsin | 12% | 100.871 | 902 | 11% |
| Q05586 NMDR1 | Trypsin | 11% | 105.373 | 938 | 11% |

As shown in figures 1 to 6 gender differences between receptor complexes have been observed.

### Discussion

The major outcome of the present invention is the use of changes in receptor complexes, especially gender-dependent derangement of receptor complexes NR1, GluR1, mAChR M1, nAChR 4 and 7, D1 and D2 in cortical samples of patients with AD. It is intriguing to find out that receptor complexes levels are different between men and women both, in controls and patients with AD. Moreover, a receptor network for the control population and the AD cohort was established by patterns and correlations that may allow interpreting the impaired wiring of the brain at the receptor level, the loss of the concerted action observed in the control cohort. The examples according to the present invention were carried out at the lowest possible post mortem period of 2hrs.

As shown in the introduction, both, nACh receptors as well as mACh receptors are involved in learning and memory and in particular in disease mechanisms of AD. Herein, M1 complex levels were increased in cortical samples of male patients with AD but not in women. This finding was never reported before and proved the biomarker nature and the nature as a pharmacological target in a male AD population. This also showed the complex deterioration of the concerted action of brain receptors in this disorder and indeed, an interaction between M1 and the NMDA receptor has been proposed already at the receptor subunit level. nAChRs 4 and 7 complex levels were both increased in AD of both sexes. A probable link between nAChR 4 and 7 and AD has been reported before in terms of co-localization of these receptor subunits in brain of patients with AD, again no nAChR complex levels have been reported.

These findings contribute to the understanding mechanisms of pathobiochemistry of AD, extend the repertoire of known potential biomarkers for AD and pharmacological modulation of the ACh system is already a therapeutic principle as mentioned above, although one may suggest to directly modulate these two nACh receptors in experimental therapies.

So far no direct interactions between M1, Nic4 and Nic7 with other brain receptors were shown. As shown in the results (figure 7) comparable electrophoretic mobility between Nic 4, Nic7 and M1 may indicate the presence of these subunits in the complexes observed. Interaction between the 5HT-1_{A}R and Nic7 were reported but in the examples of the present invention derangement of nAChRs were not accompanied by changes of 5HT-1AR complex changes. As shown in figure 7 there is no evidence for the presence of 5HT1_{A}Rs in n,mACR complexes.

Although involvement of the NMDAR is well-studied in AD no information about NRlreceptor complexes in AD brain is available. In males, NR1 receptor complex levels were significantly elevated pointing to involvement of a complex containing this subunit in AD. NR1 is mandatory for cognitive functions including learning and memory and dysregulation may well reflect memory deficits in male patients with AD, although this change may represent a byphenomenon and may be probably due to the deterioration of the orchestrated function of other receptor complex changes observed herein.

Out of the four AMPAR complexes tested, GluR1 complexes were significantly increased in brains of male AD patients. This is in agreement with increased NR1 levels that are known to regularly interact with AMPA receptors and AMPAR deficits are well-known players in cognitive function and in AD. Changes in D1 and D2 receptor complex levels are specifically preferred embodiments of the present invention: Both receptor complex levels were increased in AD in both sexes. Transmission through the G protein-coupled receptors for dopamine plays a key role in many forms of learning and memory; D1 and D2 receptors have been shown to be involved in the establishment of several forms of memory that are mediated by the cortex. D2 receptors are critical for formation and D1 receptors for extinction of fear memory in the amygdala, and D1 and D5 receptors are required for motor-skill learning in the striatum. At the cellular level, D1 receptors can enhance NMDA receptor-dependent LTP in the prefrontal cortex through activation of protein kinase A (PKA) and the D1 family of receptors plays a critical role in both LTP and LTD at corticostriatal synapses. There is broad evidence for the interaction between D1 and D2 receptors with other brain receptor systems: there is a close interaction between brain glutamatergic NMDA receptors and the monoamine dopaminergic system. Dopaminergic disturbances in the brain can lead to glutamatergic NMDA receptor changes and vice versa. NMDA receptors are mediating dopamine-glutamatergic interactions by direct coupling between both NR1 and NR2A subunits with the C terminus of dopamine D1 receptor, and dopamine depletion can change the level of NMDA receptor expression.

In the examples of the present invention using immunoprecipitation followed by mass spectrometrical identification of binding partners it was shown that D1 is a constituent of a complex with NR1, GluRl,2 and 4 (figure 10) and in male patients with AD the NR1 complex levels were increased.

It was shown that D1/D5 receptor activation results in increased cellular expression of GluR1 and incorporation at synaptic sites. Activation of dopaminergic D1 receptors in hippocampal neurons increased the pool of extrasynaptic but not synaptic AMPARs through a PKA-dependent mechanism, and their subsequent incorporation into synapses required CaMKII activity. A brief stimulation of D1 dopamine receptors, which facilitates LTP in neurons of the prefrontal cortex through a PKA-dependent mechanism, results in the clustering of GluR1-containing AMPARs near but not within the PSD. In the current study GluR1 containing complexes were increased in male patients with AD that may be in line with the abovementioned interactions.

Taken together, the present invention provides a network of brain receptor complexes for a control population and a changed network for cortical samples from AD patients as revealed by correlations between receptor systems and patterns of receptor complexes. In agreement with literature nicotinergic and muscarinic cholinergic abnormalities were observed. It was revealed that several brain receptor types were unchanged between controls and AD and changes of the dopamine receptor system were considered a novel and intriguing finding that may warrant further elucidation leading to novel experimental therapies. A D1 complex was characterised by immunoprecipitation and included NMDAR and AMPAR elements.

## Claims

1. Method for diagnosing Alzheimer's Disease (AD) post mortem, wherein the amount of at least one receptor complex, selected from the group consisting of nicotinic acetylcholine receptor complex 4, nicotinic acetylcholine receptor complex 7, dopamine receptor complex D1 and dopamine receptor complex D2, is measured in a brain sample of a patient and diagnosing AD, if the amount of the complex in the sample is increased compared to normal, non-AD levels.

2. Method for diagnosing Alzheimer's Disease (AD) in a male patient post mortem, wherein the amount of at least one receptor complex, selected from the group consisting of muscarinic acetylcholine receptor complex M1, α-amino-3-hydroxy-5-methyl-4-isoxazolepropionic acid (AMPA) receptor complex GluR1 and N-methyl-D-aspartate (NMDA) receptor complex NR1, is measured in a brain sample of a male patient and diagnosing AD, if the amount of the complex in the sample is increased compared to normal male, non-AD levels.

3. Method according to claim 1 or 2, wherein the brain sample is a temporal cortical sample.

4. Method according to any one of claims 1 to 3, wherein measuring the receptor complex includes one or more of the following steps: purification of the complex by density gradient purification, especially sucrose gradient purification, native gel electrophoresis, especially blue native polyacrylamide gel electrophoresis, immunoblotting, protein digestion, especially protein digestion with trypsin and/or chymotrypsin, and mass spectroscopy.

5. Method according to any one of claims 1 to 4, wherein the increase of the amount of the complex is at least 30%, preferably at least 50%, more preferred at least 100%, especially at least 200%, compared to healthy non-AD levels.

6. Method according to any one of claims 1 to 5, wherein measuring the receptor complex includes measuring the complexes of a molecular weight of 200 to 1500 kDa, preferably of 300 to 1000 kDa, especially of 400 to 800 kDa, in native gel electrophoresis.

7. Method according to any one of claims 1 to 6, wherein measuring the receptor complex includes identification of the receptor complexes by antibodies, especially polyclonal antibodies, against Dopamine receptor D1, Dopamine Receptor D2, Nicotinic Acetylcholine Receptor alpha4 and Nicotinic Acetylcholine Receptor alpha7; or by antibodies, especially polyclonal antibodies, against GluR1, muscarinic receptor M1 and NR1.

8. Kit for performing the method according to any one of claims 1 to 7 comprising
- detection means for at least one of nicotinic acetylcholine receptor complex 4, nicotinic acetylcholine receptor complex 7, dopamine receptor complex D1 and dopamine receptor complex D2, or at least one of muscarinic acetylcholine receptor complex M1, α-amino-3-hydroxy-5-methyl-4-isoxazolepropionic acid (AMPA) receptor complex GluR1 and N-methyl-D-aspartate (NMDA) receptor complex NR1, and
- a sample container.

9. A pharmaceutical preparation containing a D1 modulator and, optionally, a pharmaceutically acceptable excipient, for use in a method for treatment of AD.

10. Preparation according to claim 9, wherein the D1 modulator is selected from the group consisting of cis-(±)-1-(Aminomethyl)-3,4-dihydro-3-phenyl-1H-2-benzopyran-5,6-diol hydrochloride (A 68930 hydrochloride), (1R-cis)-1-(Aminomethyl)-3,4-dihydr-o-3-tricyclo[3.3.1.13,7]dec-1-yl-[1H]-2-benzopyran-5,6-diol hydrochloride (A 77636 hydrochloride), (R)-5,6,6a,7-Tetrahydro-6-methyl-4H--dibenzo[de,g]quinoline-10,11-diol hydrochloride ((R)-(-)-Apomorphine hydrochloride), (-)-(6aR,12bR)-4,6,6a,7,8,12b-Hexahydro-7-methylindolo[4,3-a] phenanthridin (CY 208-243), (±)-trans-10,11-Dihydroxy-5,6,6a,7,8-,12b-hexahydrobenzo[a]phenanthridine hydrochloride (Dihydrexidine hydrochloride), 3,4-Dihydroxyphenethylamine hydrochloride (Dopamine hydrochloride), 6-Chloro-2,3,4,5-tetrahydro-1-(4-hy-droxyphenyl)-1H-3-benzazepine-7,8-diol hydrochloride (Fenoldopam hydrochloride), (N)-1-(2-Nitrophenyl)ethylcarboxy-3-,4-dihydroxyphenethylamine (NPEC-caged-dopamine), (±)-1-Phenyl-2,3,4,5-tetrahydro-(1H)-3-benzazepine-7,8-diol hydrobromide (SKF 38393 hydrobromide), 2,3,4,5-Tetrahydro-1-phenyl-3-(2-propenyl)-1H-3-benzazepine-7,8-diol hydrobromide (SKF 77434 hydrobromide), (±)-6-Chloro-2,3,4,5-tetrahydro-1-phenyl-1H-3-benzazepine hydrobromide (SKF 81297 hydrobromide), 6-Chloro-2,3,4,5-tetrahydro-1-(3-methylphenyl)-3-(2-propenyl)-1H-3-benzazepine-7,8-di-ol hydrobromide (SKF (83822 hydrobromide), 6-Chloro-2,3,4,5-tetrahydro-3-methy-1-1-(3-methylphenyl)-1H-3-benzazepine-7,8-diol (SKF 83959 hydrobromide), 6,7,8,9,14,15-Hexahydro-7-methyl-5H--indolo[3,2-f][3]benzazecine (LE 300), (R)-(+)-7-Chloro-8-hydroxy-3-methyl-1-phenyl-2,3,4,5-tetrahydro-1H-3-benzazepine hydrochloride (SCH 23390 hydrochloride), (6aS-trans)-11-Chloro-6,6a,7,8,9,13-b-hexahydro-7-methyl-5H-benzo[d]naphth[2,1-b]azepin-12-ol hydrobromide (SCH 39166 hydrobromide), 8-Bromo-2,3,4,5-tetrahydro-3-methyl-5-phenyl-1H-3-benzazepin-7-ol hydrobromide (SKF 83566 hydrobromide), and pharmaceutically acceptable free, salt and ester forms of such D1 modulators.

11. Method for diagnosing Alzheimer's Disease (AD), wherein a D1 modulator radiolabelled with a positron-emitting radionuclide is administered to a patient, the patient's brain is subjected to a Positron emission tomography (PET) and wherein an increased binding and localisation of the radiolabelled D1 modulator in the brain compared to a normal, non-AD person, is indicative of AD.

12. Method according to claim 11, wherein the radionuclide is selected from carbon-11, nitrogen-13, oxygen-15, fluorine-18, and rubidum-82.

13. Method according to claim 11 or 12, wherein the patient's temporal cortex is subjected to PET.
